(19)

Europäisches
Patentamt
European
Patent Office
Office européen
des brevets

(11) **EP 1 939 286 A1**

(12) **EUROPEAN PATENT APPLICATION**
published in accordance with Art. 153(4) EPC

(43) Date of publication:
**02.07.2008   Bulletin 2008/27**

(21) Application number: **06781879.9**

(22) Date of filing: **28.07.2006**

(51) Int Cl.:
*C12N 15/09* (2006.01)          *A61K 38/00* (2006.01)
*A61K 38/22* (2006.01)          *A61P 9/10* (2006.01)
*A61P 43/00* (2006.01)          *C07K 7/08* (2006.01)
*C07K 14/475* (2006.01)         *C07K 16/24* (2006.01)
*C12N 1/15* (2006.01)           *C12N 1/19* (2006.01)
*C12N 1/21* (2006.01)           *C12N 5/10* (2006.01)
*C12P 21/02* (2006.01)          *C12Q 1/02* (2006.01)
*G01N 33/15* (2006.01)          *G01N 33/50* (2006.01)

(86) International application number:
**PCT/JP2006/314969**

(87) International publication number:
**WO 2007/013586 (01.02.2007 Gazette 2007/05)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **29.07.2005   JP 2005221635**

(71) Applicants:
• **KYOWA HAKKO KOGYO CO., LTD.
  Chiyoda-ku,
  Tokyo 100-8185 (JP)**
• **JAPAN AS REPRESENTED BY THE PRESIDENT
  OF NATIONAL
  CARDIOVASCULAR CENTER
  Suita-shi, Osaka 565-8565 (JP)**
• **OSAKA UNIVERSITY
  Suita-shi, Osaka 565-0871 (JP)**

(72) Inventors:
• **YAMASAKI, Motoo,
  c/o Kyowa Hakko Kogyo Co.,Ltd.
  Machida-shi, Tokyo 1948533 (JP)**

• **TAKAHASHI, Noriyuki,
  c/o Kyowa Hakko Kogyo Co.,Ltd.
  Machida-shi, Tokyo 1948533 (JP)**
• **MINAMINO, Naoto,
  c/o National Cardiovascular Center
  Suita-shi, Osaka 5658565 (JP)**
• **SASAKI, Kazuki,
  c/o National Cardiovascular Center
  Suita-shi, Osaka 5658565 (JP)**
• **TAKAO, Toshifumi,
  c/o Osaka University
  Suita-shi, Osaka 5650871 (JP)**
• **SATOMI, Yoshinori
  Suita-shi, Osaka 5650875 (JP)**

(74) Representative: **Vossius & Partner
  Siebertstrasse 4
  81675 München (DE)**

(54) **NOVEL PEPTIDES**

(57)   The present invention provides novel peptides with circulation-modulating activity. These peptides are useful as circulation-modulating agents and vasopressors because of their circulation-modulating activity, and can be used for treating diseases of the circulatory system such as myocardial infarction, ischemic heart disease, cerebral infarction, or the like.

**EP 1 939 286 A1**

**Description**

Technical Field

The present invention relates to novel peptides, DNAs encoding these peptides, antibodies that specifically bind to these peptides, methods for producing these peptides, pharmaceuticals comprising these peptides, and methods of using these peptides for screening.

Background Art

The VGF gene was identified as a gene that increases in rat PC12 cells stimulated with nerve growth factor (see Non-Patent Document 1), which in turn led to the isolation of a human VGF gene(see Non-Patent Document 2). In VGF gene-disrupted mice, food consumption remained the same, but a decrease in body weight and body fat, an increase in oxygen consumption and locomoter activity, and abnormal reproductive functions were observed. In particular, enhanced energy metabolism was observed (see Non-Patent Document 3).
VGF genes are expressed in the central and peripheral nervous systems, as well as in endocrine and neuroendocrine cells. Their expression and distribution are similar to the expression patterns of neuropeptide Y, peptide YY, ghrelin, cholecystokinin, and the like which regulate feeding behavior and the gastrointestinal motility; and they are present in parts that are related to energy metabolism (see Non-Patent Document 4).
Proteins encoded by the VGF genes (hereinafter referred to as VGFs) comprise 615 amino acids in humans and 617 amino acids in rats/mice. Amino acids 1 to 22 of VGF is a signal peptide, and there are sequences processed by amidation, cleaved by prohormone convertase, or the like. Processing of VGF has been investigated mainly in rats, and the following peptides derived from VGF have been found in rat brains: rat VGF (598-617), rat VGF (599-617), rat VGF (601-617), rat VGF (602-617), rat VGF (587-617), rat VGF (588-617), rat VGF (567-617), rat VGF (556-617), rat VGF (489-617), and rat VGF18 (18 kDa: unknown sequence) (see Non-Patent Document 5; the numbers in the parentheses indicate the positions of the peptide sequences in the VGF amino acid sequence). Direct administration of partial peptides of rat VGF (556-617): rat VGF (577-617), rat VGF (588-617), and rat VGF (599-617) to the paraventricular nucleus of the hypothalamus (PVN) of male rats showed an erection inducing activity, but this activity was not observed with rat VGF (556-576) (see Non-Patent Document 6). Furthermore, it is reported that when VGF (588-596) was administered intraperitoneally to VGF gene-disrupted mice, the body weight increased by about 10% to 15% (see Patent Document 1).
The following peptides are derived from human VGF and present in the human cerebrospinal fluid: human VGF (23-62), human VGF (23-59), and human VGF (26-62) (see Non-Patent Document 7); and human VGF (23-58), human VGF (24-59), human VGF (24-62), human VGF (26-57), human VGF (26-58), human VGF (26-59), human VGF (26-61), human VGF (26-64), human VGF (49-62), human VGF (90-114), human VGF (350-367), human VGF (350-370), human VGF (373-404), human VGF (373-417), human VGF (420-471), and human VGF (420-478) (see Patent Document 2). Furthermore, a peptide whose sequence corresponds to rat VGF (588-617) and is identical to positions 586-615 of the human VGF sequence has been isolated from the bovine posterior pituitary gland (see Non-Patent Document 8). However, there has been no report on the physiological activities of these peptides derived from human or bovine VGF.
As antibodies that specifically recognize VGF or peptides derived from VGF, polyclonal antibodies against antigenic peptide comprising the C-terminal 573-617th amino acid sequence of rat VGF (see Non-Patent Document 9), polyclonal antibodies against antigenic peptide comprising the 556-565th amino acid sequence of human VGF (see Non-Patent Document 4), polyclonal antibodies against antigenic peptide comprising the 443-588th amino acid sequence of rat VGF (see Non-Patent Document 10), and the like have been reported.

[Patent Document 1] WO01/07477
[Patent Document 2] WO02/82075
[Non-Patent Document 1] Science, (USA), 1985, Vol. 229, No. 4711, pp. 393-395.
[Non-Patent Document 2] Genomics, (USA), 1997, Vol. 45, No. 2, pp. 443-446.
[Non-Patent Document 3] Neuron, (USA), 1999, Vol. 23, No. 3, pp. 537-548.
[Non-Patent Document 4] Cellular and Molecular Neurobiology, (USA), 2004, Vol. 24, No. 4, pp. 517-533.
[Non-Patent Document 5] Journal ofNeurochemistry, (UK), 2002, Vol. 81, No. 3, pp. 565-574.
[Non-Patent Document 6] European Journal of Neuroscience, (France), 2004, Vol. 20, No. 11, pp. 3035-3040.
[Non-Patent Document 7] Journal of Chromatography B: Biomedical Sciences and Applications, (Holland), 2001, Vol. 754, No. 2, pp. 357-367.
[Non-Patent Document 8] Endocrinology, (USA), 1994, Vol. 135, No. 6, pp. 2742-2748.
[Non-Patent Document 9] Endocrinology, (USA), 1999, Vol. 140, No. 8, pp. 3727-3735.
[Non-Patent Document 10] The EMBO Journal, (UK), 1989, Vol. 8, No.8, pp. 2217-2223.

Disclosure of the Invention

[Problems to be Solved by the Invention]

An objective of the present invention is to provide novel peptides having a circulation-modulating activity, as well as to provide DNAs encoding these peptides, antibodies that specifically bind to these peptides, methods for producing these peptides, pharmaceuticals comprising these peptides, and methods of screening using these peptides.

[Means for Solving the Problems]

The present invention relates to the following [1] to [12] :

[1] a peptide of any one of (a) to (e) below or a pharmaceutically acceptable salt thereof:

(a) a peptide comprising the amino acid sequence of any one of SEQ ID NOS: 1 to 4, 28 and 29 (but excluding peptides comprising the amino acid sequence of SEQ ID NOS: 9, 30, 31, 32 or 33);
(b) a peptide comprising an amino acid sequence with substitution, deletion, or addition of one to five amino acids in the amino acid sequence of any one of SEQ ID NOS: 1 to 4, 28 and 29, wherein the peptide has an activity of increasing the intracellular calcium ion concentration of cardiac or vascular cells;
(c) a peptide comprising an amino acid sequence having 90% or higher homology to the amino acid sequence of any one of SEQ ID NOS: 1 to 4, 28 and 29, wherein the peptide has an activity of increasing the intracellular calcium ion concentration of cardiac or vascular cells;
(d) a peptide represented by the following formula (I) (but excluding peptides comprising the sequences of SEQ ID NOS: 14 to 16),

$$Z^1\text{-A-}Z^2 \qquad \text{(I)}$$

(wherein, $Z^1$ represents a hydrogen atom or a peptide residue of any sequence comprising one to eleven amino acids, A represents a peptide residue comprising the amino acid sequence of SEQ ID NO: 17, and $Z^2$ represents an amino group or a peptide residue of any sequence comprising one to 38 amino acids); and
(e) a peptide represented by the following formula (II)

$$R^1\text{-B-}R^2 \qquad \text{(II)}$$

(wherein, $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and B represents a peptide residue of the peptide of any one of the above-mentioned (a) to (d));

[2] the peptide of [1]or a pharmaceutically acceptable salt thereof, wherein the peptide is a peptide of any one of (a) to (f):

(a) a peptide comprising the amino acid sequence of SEQ ID NO: 1;
(b) a peptide comprising the amino acid sequence of SEQ ID NO: 2;
(c) a peptide comprising the amino acid sequence of SEQ ID NO: 28;
(d) a peptide comprising the amino acid sequence of SEQ ID NO: 29;
(e) a peptide wherein in formula (II) of [1], $R^1$ is a hydrogen atom, B is a peptide residue comprising the amino acid sequence of SEQ ID NO: 3, and $R^2$ is unsubstituted amino; and
(f) a peptide wherein in formula (II) of [1], $R^1$ is a hydrogen atom, B is a peptide residue comprising the amino acid sequence of SEQ ID NO: 4, and $R^2$ is unsubstituted amino;

[3] a DNA encoding any one of the peptides of (a) to (c) of [1];
[4] a recombinant vector obtainable by incorporating the DNA of [3] into a vector;
[5] a transformant obtainable by introducing the recombinant vector of [4] into a host cell;
[6] a method for producing a peptide, which comprises culturing the transformant of [5] in a medium so as to produce and accumulate said peptide in the culture, and recovering said peptide from the culture;
[7] an antibody that binds to an epitope present in the amino acid sequence of SEQ ID NO: 4 or a sequence wherein the C terminus of the amino acid sequence of SEQ ID NO: 4 is amidated;

[8] a method of detecting or quantifying the peptide of [1] or [2], which comprises using the antibody of [7];

[9] a circulation-modulating agent comprising as an active ingredient at least one peptide selected from (a) to (f) below or a pharmaceutically acceptable salt thereof:

(a) a peptide comprising the amino acid sequence of any one of SEQ ID NOS: 1 to 5, 28 and 29;

(b) a peptide comprising an amino acid sequence with substitution, deletion, or addition of one to five amino acids in the amino acid sequence of any one of SEQ ID NOS: 1 to 5, 28 and 29, wherein the peptide has an activity of increasing the intracellular calcium ion concentration of cardiac or vascular cells;

(c) a peptide comprising an amino acid sequence having 90% or higher homology to the amino acid sequence of any one of SEQ ID NOS: 1 to 5, 28 and 29, wherein the peptide has an activity of increasing the intracellular calcium ion concentration of cardiac or vascular cells; and

(d) a peptide represented by formula (I) of [1];

(e) a peptide comprising the amino acid sequence of SEQ ID NO: 23, or a peptide comprising an amino acid sequence wherein any amino acid sequence comprising one to 32 amino acids has been added to the N terminus of the amino acid sequence of SEQ ID NO: 23;

(f) a peptide represented by the following formula (III)

$$R^3\text{-}C\text{-}R^4 \qquad (III)$$

(wherein, $R^3$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^4$ represents hydroxy, substituted or unsubstituted alkoxy, or substituted or unsubstituted amino; and C represents a peptide residue of the peptide of any one of the above-mentioned (a) to (e));

[10] a method of screening for a substance that inhibits peptide-induced increase of intracellular calcium ion concentration in cardiac or vascular cells, which comprises:

measuring the cellular response elicited when a test substance and the peptide of any one of (a) to (f) of [9] or a pharmaceutically acceptable salt thereof are contacted with cardiac or vascular cells; and

identifying the test substance as a substance that inhibits the peptide-induced increase of intracellular calcium ion concentration in cardiac or vascular cells, if the test substance suppresses the cellular response compared to the cellular response when said peptide or a pharmaceutically acceptable salt thereof is contacted with said cells in the absence of the test substance;

[11] a method of screening for a substance that promotes peptide-induced increase of intracellular calcium ion concentration in cardiac or vascular cells, which comprises:

measuring the cellular response elicited when a test substance and the peptide of any one of (a) to (f) of [9] or a pharmaceutically acceptable salt thereof are contacted with cardiac or vascular cells; and

identifying the test substance as a substance that promotes the peptide-induced increase of intracellular calcium ion concentration in cardiac or vascular cells, if the test substance promotes the cellular response as compared to the cellular response when said peptide or a pharmaceutically acceptable salt thereof is contacted with said cells in the absence of the test substance, and

[12] a method of screening for a peptide receptor agonist or antagonist, the method comprising:

measuring the binding level of said peptide or a pharmaceutically acceptable salt thereof to cardiac or vascular cells, or a membrane fraction of said cells, when a test substance and the peptide of any one of (a) to (f) of [9] or a pharmaceutically acceptable salt thereof are contacted with said cells or cell membrane fraction; and

identifying the test substance as an agonist or antagonist for the receptor of said peptide if the test substance causes a decrease in the binding level of said peptide or a pharmaceutically acceptable salt thereof as compared to when said peptide or a pharmaceutically acceptable salt thereof is contacted with said cells or cell membrane fraction in the absence of the test substance.

[Effects of the Invention]

The present invention provides novel peptides having circulation-modulating activity, DNAs encoding the peptides, antibodies that specifically bind to these peptides, pharmaceuticals comprising these peptides, methods for producing these peptides, and methods that use these peptides for screening for substances that promote or suppress the activity of these peptides, agonists or antagonists for the receptors of these peptides. The peptides of the present invention are useful for treating diseases of the circulatory system, such as myocardial infarction, ischemic heart disease, cerebral infarction, and the like.

Brief Description of the Drawings

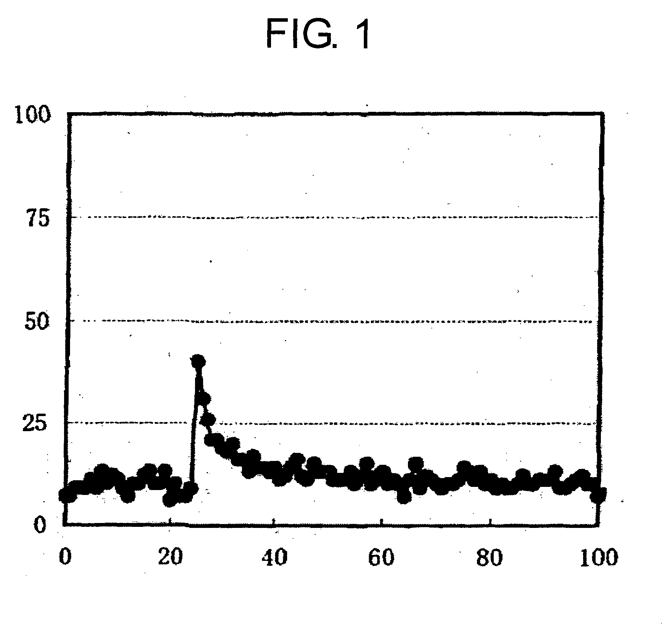

Fig. 1 shows an increase of intracellular calcium concentration in cardiac cells of the apoaequorin-expressing mice due to 1 $\mu$mol/L of Peptide 1 (SEQ ID NO: 27). The horizontal axis indicates the time (seconds) after addition of the medium, and the vertical axis indicates the relative luminescence unit (RLU) per second. Peptide 1 was added at 25 seconds.

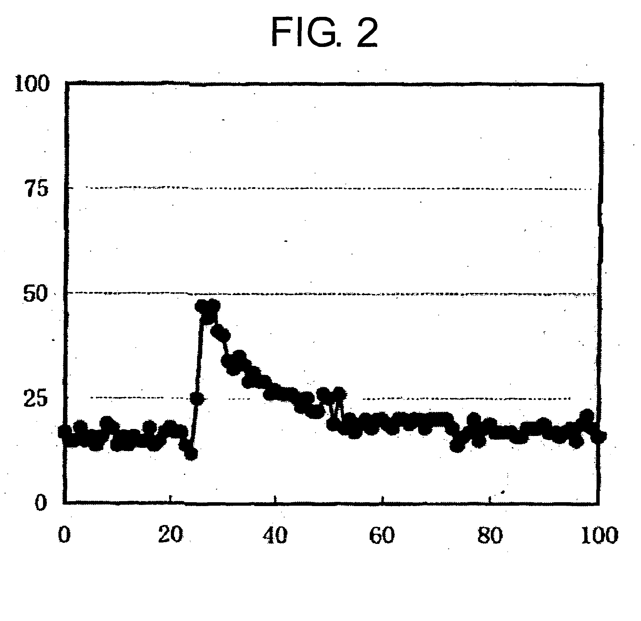

Fig. 2 shows an increase of intracellular calcium concentration in vascular cells of the apoaequorin-expressing mice due to 5 $\mu$mol/L of Peptide 2 (SEQ ID NO: 26). The horizontal axis indicates the time (seconds) after addition of the medium, and the vertical axis indicates the relative luminescence unit (RLU) per second. Peptide 2 was added at 25 seconds.

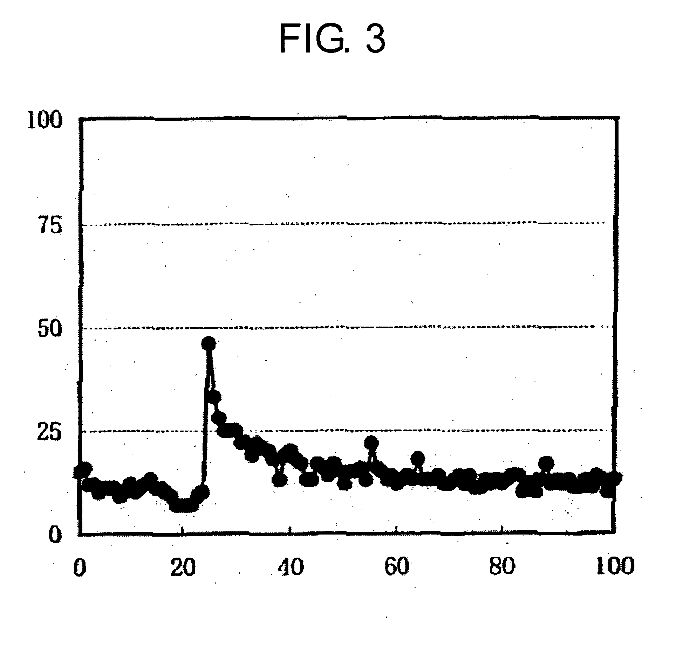

Fig. 3 shows an increase of intracellular calcium concentration in cardiac cells of the apoaequorin-expressing mice due to 1 $\mu$mol/L of Peptide 2. The horizontal axis indicates the time (seconds) after addition of the medium, and the vertical axis indicates the relative luminescence unit (RLU) per second. Peptide 2 was added at 25 seconds.

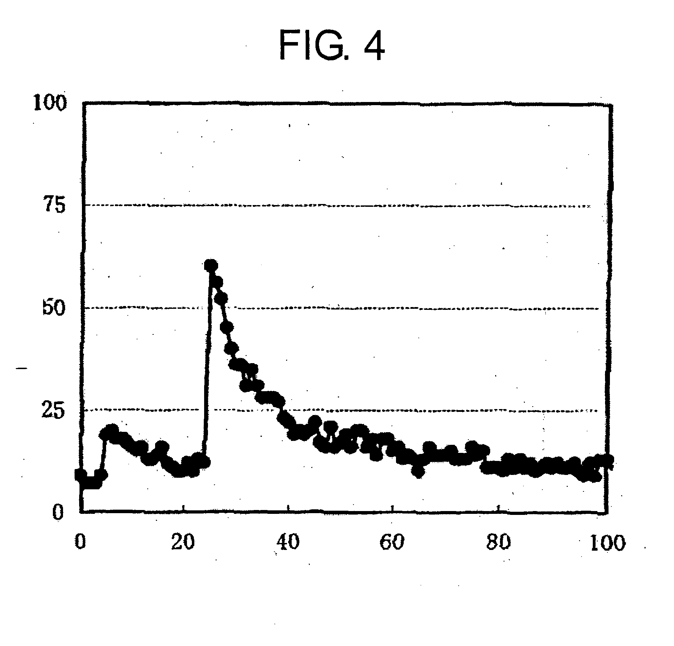

Fig. 4 shows an increase of intracellular calcium concentration in cardiac cells of the apoaequorin-expressing mice due to 1 $\mu$mol/L of Peptide 3 (SEQ ID NO: 2). The horizontal axis indicates the time (seconds) after addition of the medium, and the vertical axis indicates the relative luminescence unit (RLU) per second. Peptide 3 was added at 25 seconds.

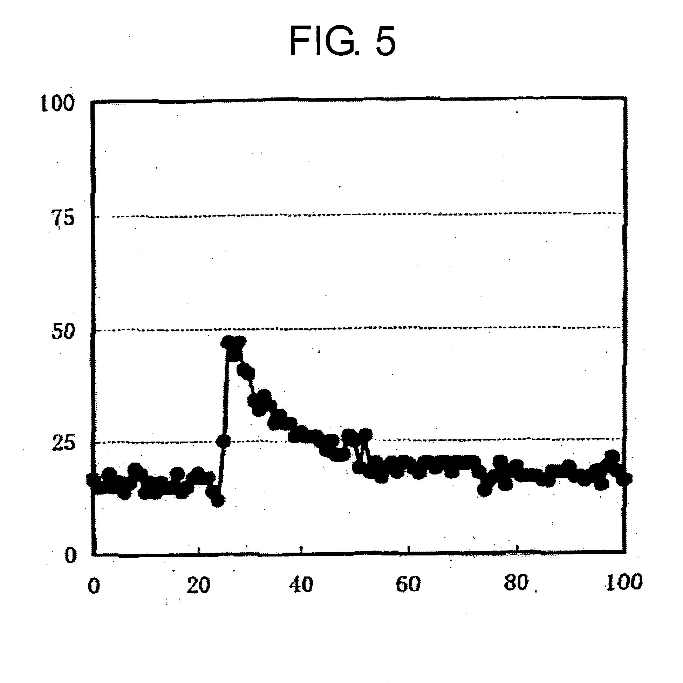

Fig. 5 shows an increase of intracellular calcium concentration in vascular cells of the apoaequorin-expressing mice due to 5 $\mu$mol/L of Peptide 4 (SEQ ID NO: 5). The horizontal axis indicates the time (seconds) after addition of the medium, and the vertical axis indicates the relative luminescence unit (RLU) per second. Peptide 4 was added at 25 seconds.

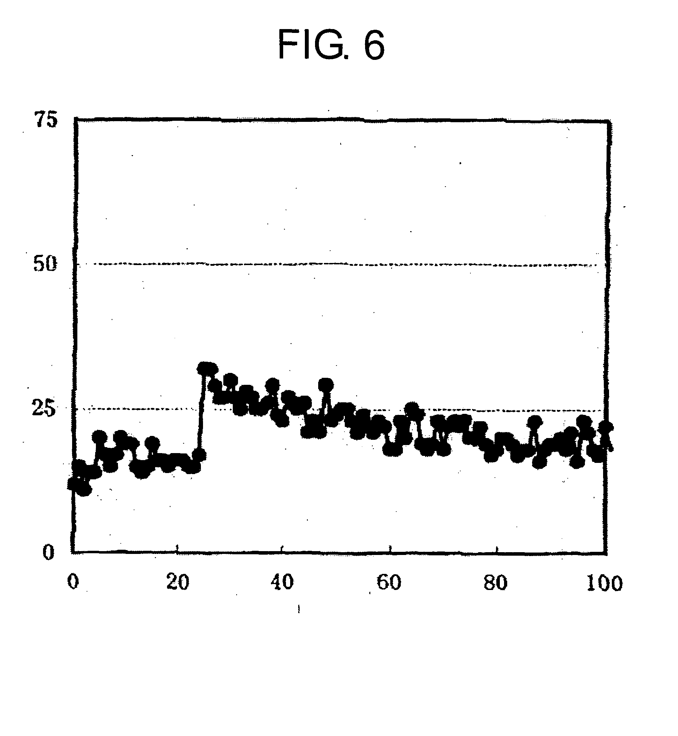

Fig. 6 shows an increase of intracellular calcium concentration in cardiac cells of the apoaequorin-expressing mice due to 5 $\mu$mol/L of Peptide 5 (SEQ ID NO: 1). The horizontal axis shows the time (seconds) after addition of the medium, and the vertical axis indicates the relative luminescence unit (RLU) per second. Peptide 5 was added at 25 seconds.

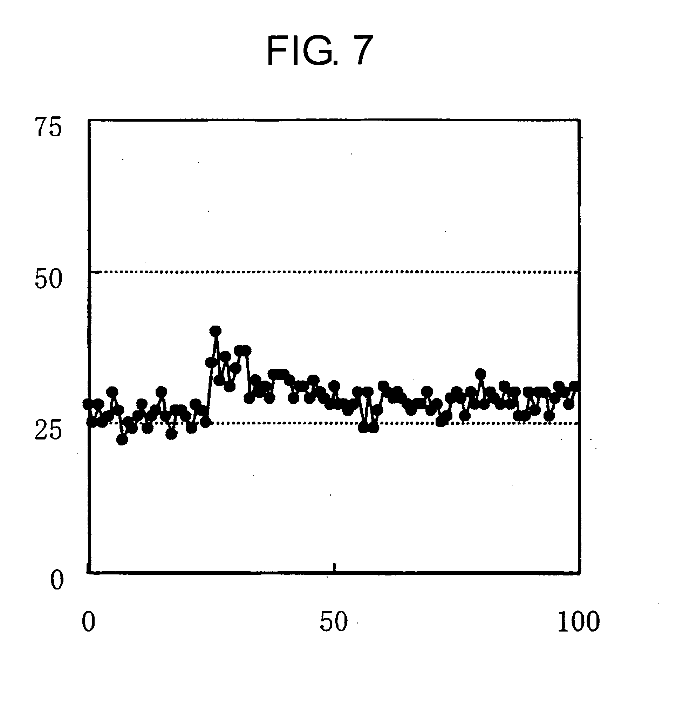

Fig. 7 shows an increase of intracellular calcium concentration in vascular cells of the apoaequorin-expressing mice due to 5 $\mu$mol/L of Peptide 9 (SEQ ID NO: 28). The horizontal axis indicates the time (seconds) after addition of the medium, and the vertical axis indicates the relative luminescence unit (RLU) per second. Peptide 9 was added at 25 seconds.

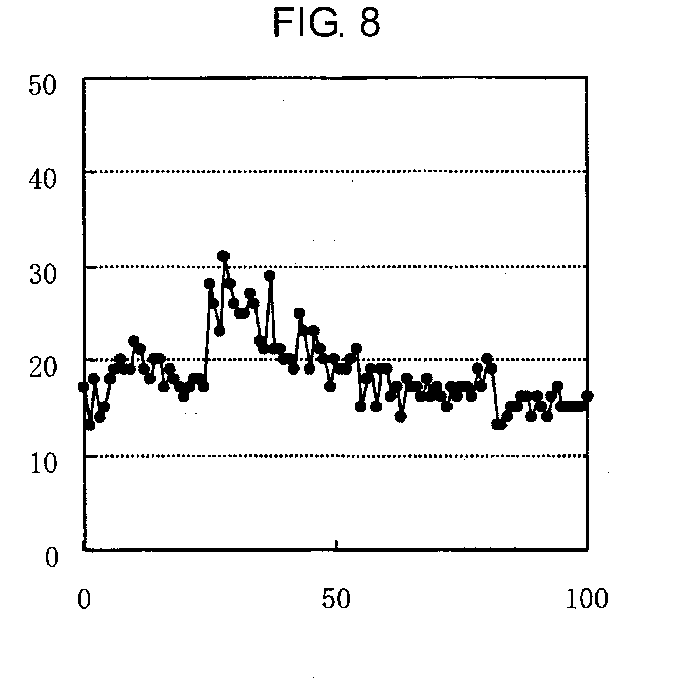

Fig. 8 shows an increase of intracellular calcium concentration in cardiac cells of the apoaequorin-expressing mice due to 1 $\mu$mol/L of Peptide 10 (SEQ ID NO: 29). The horizontal axis indicates the time (seconds) after addition of the medium, and the vertical axis indicates the relative luminescence unit (RLU) per second. Peptide 10 was added at 25 seconds.

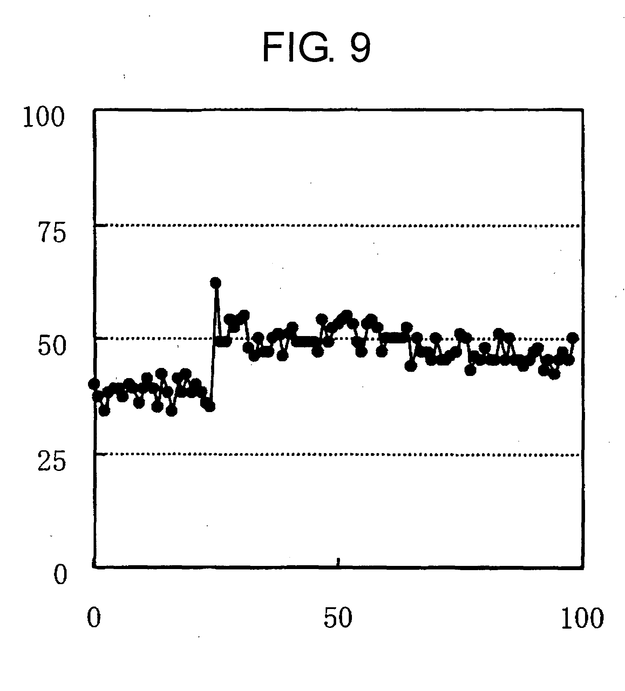

Fig. 9 shows an increase in the intracellular calcium concentration in vascular cells of the apoaequorin-expressing mice due to 5 $\mu$mol/L of Peptide 10. The horizontal axis indicates the time (seconds) after addition of the medium, and the vertical axis indicates the relative luminescence units (RLU) per second. Peptide 10 was added at 25 seconds.

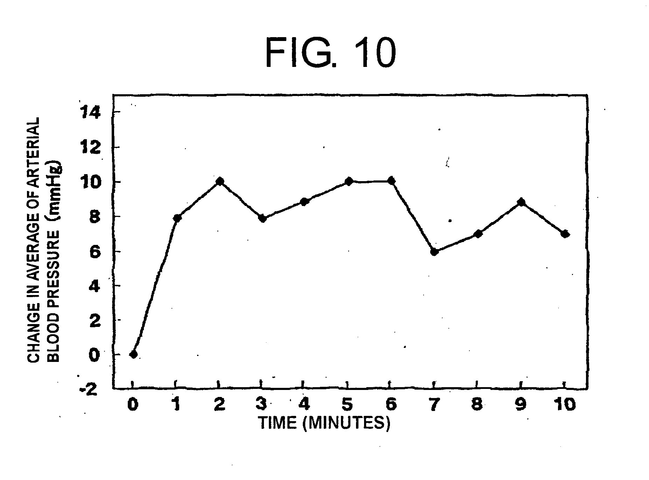

Fig. 10 shows an increase of blood pressure of rats due to Peptide 2. The horizontal axis indicates the time (minutes) after Peptide 2 administration, and the vertical axis indicates the change in average arterial blood pressure.

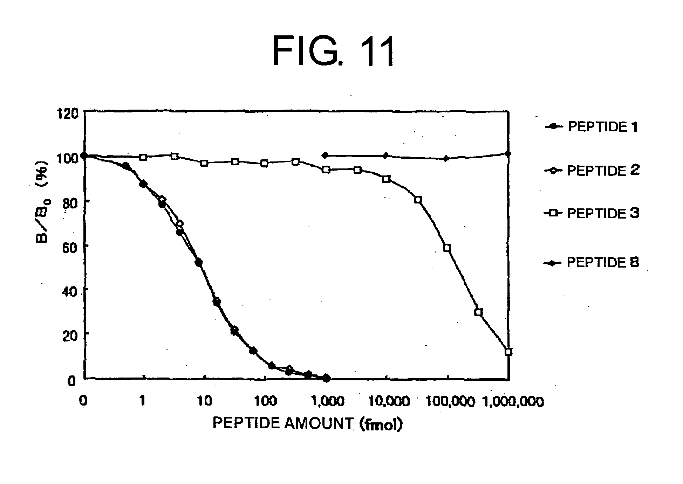

Fig. 11 shows binding specificity of antiserum to various peptides derived from VGF. The ratios of the specific binding with respect to the maximum binding level of the antiserum when adding Peptide 1, Peptide 2, Peptide 3, and Peptide 8 (SEQ ID NO: 8) are indicated by closed circle, opened diamond, open square, and closed diamond, respectively. The horizontal axis indicates the amount (fmol) of each peptide added, and the vertical axis shows the percentage of specific binding with respect to the maximum binding level, $B/B_0$ (%).

Best Mode for Carrying Out the Invention

1. Peptides of the present invention

A peptide of the present invention is a peptide of any one of the following (a) to (e), or a pharmaceutically acceptable salt thereof:

(a) A peptide comprising the amino acid sequence shown in any one of SEQ ID NOS: 1 to 4, 28 and 29 (but excluding peptides comprising the amino acid sequence shown in SEQ ID NOS: 9, 30, 31, 32 and 33).
The amino acid sequence shown in SEQ ID NO: 9 is a human VGF amino acid sequence. The amino acid sequence of the peptide of (a) may consist of any number of amino acids so long as it comprises the sequence shown in any of SEQ ID NOS: 1 to 4, 28 and 29, but is preferably 80 or less, more preferably 60 or less, or particularly preferably 40 or less.
(b) A peptide comprising an amino acid sequence with substitution, deletion, or addition of one to five amino acids in the amino acid sequence shown in any of SEQ ID NOS: 1 to 4, 28 and 29, wherein the peptide has an activity of increasing the intracellular calcium ion concentration of cardiac or vascular cells.
(c) A peptide comprising an amino acid sequence having 90% or higher homology to the amino acid sequence shown in any of SEQ ID NOS: 1 to 4, 28 and 29, wherein the peptide has an activity of increasing the intracellular calcium ion concentration of cardiac or vascular cells.
(d) A peptide represented by the following formula (I) (but excluding peptides comprising the sequences shown in SEQ ID NOS: 14 to 16),

$$Z^1\text{-}A\text{-}Z^2 \qquad (I)$$

(wherein, $Z^1$ represents a hydrogen atom or a peptide residue of any sequence comprising one to eight amino acids, A represents a peptide residue comprising the amino acid sequence shown in SEQ ID NO: 17, and $Z^2$ represents an amino group or a peptide residue of any sequence comprising one to 32 amino acids).
The amino acid sequences shown in SEQ ID NOS: 14 to 16 are the amino acid sequences of rat VGF (567-585), rat VGF (567-617), and rat VGF (556-617), respectively.
(e) A peptide represented by the following formula (II)

$$R^1\text{-}B\text{-}R^2 \qquad (II)$$

(wherein, $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and B represents a peptide residue of the peptide of any one of the above-mentioned (a) to (d)).

The phrase "substitution, deletion, or addition of one to five amino acid residues in the amino acid sequence shown in any of SEQ ID NOS: 1 to 4, 28 and 29" means that one to five amino acid substitutions, deletions, or additions are present at any of one or more positions in the same amino acid sequences, and the substitutions, deletions, or additions may occur simultaneously. Examples of amino acids that are substituted or added include the 20 L-amino acids known as essential amino acids, which are specifically, L-alanine, L-asparagine, L-aspartic acid, L-arginine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, and L-cysteine, but are not limited thereto; and for example, other amino acids such as *tert*-leucine, norleucine, norvaline, 2-aminobutanoic acid, *O*-methylserine, *t*-butylglycine, t-butylalanine, cyclohexylalanine, isoaspartic acid, isoglutamic acid, 2-aminoadipic acid, 2-aminosuberic acid, ornithine, 2,4-diaminobutanoic acid, 2,3-diaminopropionic acid, 3-hydroxyproline, 4-hydroxyproline, homoserine, D-amino acids and β-amino acids may be included.
Examples of amino acid residues that can be mutually substituted are shown below. Amino acid residues included in the same group can be mutually substituted.

Group A: leucine, isoleucine, norleucine, valine, norvaline, alanine, 2-aminobutanoic acid, methionine, *O*-methylserine, t-butylglycine, t-butylalanine, cyclohexylalanine, *tert*-leucine
Group B: aspartic acid, glutamic acid, isoaspartic acid, isoglutamic acid, 2-aminoadipic acid, 2-aminosuberic acid
Group C: asparagine, glutamine
Group D: lysine, arginine, ornithine, 2,4-diaminobutanoic acid, 2,3-diaminopropionic acid

Group E: proline, 3-hydroxyproline, 4-hydroxyproline
Group F: serine, threonine, homoserine
Group G: phenylalanine, tyrosine

The phrase "amino acid sequence having 90% or higher homology to the amino acid sequence shown in any of SEQ ID NOS: 1 to 4, 28 and 29" refers to an amino acid sequence having 90% or higher, or preferably 95% or higher homology (the number of identical amino acids between the sequence of interest and the sequence of any one of SEQ ID NOS: 1 to 4, 28 and 29 with which homology analysis was performed/ (total number of amino acids of the sequence of any one of SEQ ID NOS: 1 to 4, 28 and 29 with which homology analysis was performed + number of gaps inserted during the alignment)) when alignment is performed by calculation using a homology analysis program BLAST 2 Sequences (FEMS Microbiol Lett. 174, 247 (1999)) under default settings (program: blastp; matrix: BLOSUM62; open gap: 11 penalties; extension gap: I penalty; gap x_dropoff: 50; expect: 10.0; word size: 3).

In equation (I), A is the amino acid sequence shown in SEQ ID NO: 17, or more specifically, a peptide residue comprising the amino acid sequence represented by the following formula (IV).

$$\text{His-}X^1\text{-His-His-Ala-Leu-Pro-Pro-}X^2\text{-Arg-His-}X^3\text{-Pro} \qquad \text{(IV)}$$

(wherein, $X^1$, $X^2$ and $X^3$ independently represent the same or different amino acids) In formula (IV), $X^1$, $X^2$ and $X^3$ independently represent the same or different amino acids, but preferably $X^1$ is tyrosine or phenylalanine, $X^2$ is serine, threonine, homoserine, leucine, isoleucine, norleucine, valine, norvaline, alanine, 2-aminobutanoic acid, methionine, *O*-methylserine, t-butylglycine, *t*-butylalanine, cyclohexylalanine or *tert*-leucine, and $X^3$ is tyrosine, phenylalanine or histidine, more preferably $X^2$ is serine or alanine, and $X^3$ is tyrosine or histidine, and even more preferably $X^1$, $X^2$ and $X^3$ are tyrosine, serine and tyrosine, respectively, or phenylalanine, alanine and histidine, respectively.

In formula (I) $Z^1$ is a hydrogen atom or a peptide residue of any sequence comprising one to eleven amino acids, but is preferably a hydrogen atom or a peptide residue comprising the amino acid sequence represented by the following formula (V) (SEQ ID NO: 18).

$$\text{Thr-Leu-Gln-Pro-Pro-}X^4\text{-}X^5\text{-}X^6\text{-Arg-Arg-Arg} \qquad \text{(V)}$$

(wherein, $X^4$, $X^5$ and $X^6$ independently represent the same or different amino acids)
In formula (V), $X^4$, $X^5$ and $X^6$ are preferably serine, threonine, homoserine, leucine, isoleucine, norleucine, valine, norvaline, alanine, 2-aminobutanoic acid, methionine, *O*-methylserine, t-butylglycine, t-butylalanine, cyclohexylalanine or *tert*-leucine, more preferably $X^4$ and $X^5$ are serine or alanine and $X^6$ is serine or leucine, and even more preferably $X^4$, $X^5$ and $X^6$ are serine, alanine and leucine, respectively, or alanine, serine and serine, respectively.

In formula (I), $Z^2$ is an amino group or a peptide residue of any sequence comprising one to 38 amino acids, but is preferably an amino group, a peptide residue comprising the amino acid sequence represented by the following formula (VI) (SEQ ID NO: 19), or a peptide residue comprising an amino acid sequence represented by the following formula (VII) (SEQ ID NO: 20).

$$X^7\text{-}X^8\text{-Glu-Ala-Gln-Ala} \qquad \text{(VI)}$$

(wherein, $X^7$ and $X^8$ independently represent the same or different amino acids)
$X^9$-$X^{10}$-Glu-Ala-Gln-Ala-Arg- Arg- Ala-Gln-Glu-Glu-Ala-$X^{11}$-Ala-Glu-Glu-Arg-Arg-Leu-Gln-Glu-Gln-Glu-Glu-Leu-Glu-Asn-Tyr-Ile-Glu-His-Val-Leu-Leu-$X^{12}$-Arg-Pro (VII) (wherein, $X^9$, $X^{10}$, $X^{11}$ and $X^{12}$ independently represent the same or different amino acids)

In formulas (VI) and (VII), preferably, $X^7$ and $X^9$ are glycine, aspartic acid, glutamic acid, isoaspartic acid, isoglutamic acid, 2-aminoadipic acid or 2-aminosuberic acid, $X^8$ and $X^{10}$ are arginine, lysine, ornithine, 2,4-diaminobutanoic acid, 2,3-diaminopropionic acid, leucine, isoleucine, norleucine, valine, norvaline, alanine, 2-aminobutanoic acid, methionine, *O*-methylserine, t-butylglycine, t-butylalanine, cyclohexylalanine or *tert*-leucine, $X^{11}$ is aspartic acid, glutamic acid, isoaspartic acid, isoglutamic acid, 2-aminoadipic acid or 2-aminosuberic acid, $X^{12}$ is arginine, lysine, ornithine, 2,4-diaminobutanoic acid, 2,3-diaminopropionic acid or histidine, more preferably, $X^7$ and $X^9$ are glycine or aspartic acid, $X^8$ and $X^{10}$ are arginine or leucine, $X^{11}$ is aspartic acid or glutamic acid, and $X^{12}$ is arginine or histidine, and even more preferably $X^7$ and $X^8$ are glycine and arginine respectively, or aspartic acid and leucine respectively, $X^9$, $X^{10}$, $X^{11}$ and $X^{12}$ are glycine, arginine, glutamic acid and arginine, respectively, or aspartic acid, leucine, aspartic acid and histidine, respectively.

In the definition of each group of the above-mentioned formula (II), examples of alkanoyl include a straight chain or branched chain alkanoyl comprising one to twenty carbon atoms, such as formyl, acetyl, propionyl, butyryl, isobutyryl, valeryl, isovaleryl, pivaloyl, hexanoyl, heptanoyl, lauroyl, eicosanoyl, and the like.

Examples of the aryl moiety of aroyl and aryloxycarbonyl include phenyl, naphthyl, and the like, and comprise six to 15 carbons.

Examples of the heteroaryl moiety of heteroarylcarbonyl and heteroaryloxycarbonyl include furyl, thienyl, pyrrolyl, pyrazolyl, imidazolyl, oxazolyl, thiazolyl, pyridyl, pyridazinyl, pyrimidinyl, pyrazinyl, indolyl, indazolyl, benzimidazolyl, quinolyl, isoquinolyl, cinnolinyl, quinazolynyl, quinoxalinyl, naphthylidinyl, and the like.

Examples of the alkyl moiety of alkoxycarbonyl and alkoxy include a straight chain or branched chain alkyl moiety of one to twenty carbons such as methyl, ethyl, propyl, isopropyl, butyl, pentyl, hexyl, heptyl, decyl, dodecyl, eicosyl, and the like.

Examples of the substituents of the substituted alkanoyl, substituted alkoxycarbonyl and substituted alkoxy, which may be the same or different and in number of 1 to 3, include hydroxy; carboxy; aliphatic cyclic alkyl of three to eight carbons including cyclopropyl, cyclobutyl, cyclopentyl, cyclohexyl, cycloheptyl, cyclooctyl and the like; substituted and unsubstituted phenyl; substituted and unsubstituted fluorenyl, and the like. Examples of the substituents of the substituted phenyl, which may be the same or different and in number of 1 to 3, include alkyl, alkoxy, hydroxy, nitro, sulfo, cyano, halogen, and the like, and examples of the halogen include each of fluorine, chlorine, bromine, and iodine atoms. The alkyl moiety of alkyl and alkoxy serving as substituents of the substituted phenyl has the same meaning as the alkyl moiety of the aforementioned alkoxycarbonyl and alkoxy.

There are one to three same or different substituents in the substituted aroyl, substituted aryloxycarbonyl, substituted heteroarylcarbonyl and substituted heteroaryloxycarbonyl; and they have the same meanings as the above-mentioned substituents of the substituted phenyl.

One to two substituents in the substituted amino which are the same or different include, for example, substituted or unsubstituted alkyl, substituted or unsubstituted aryl, or the like. Alkyl has the same meaning as the aforementioned alkyl moiety of alkoxy or the like, and substituents of the substituted alkyl have the same meaning as the aforementioned substituents of the substituted alkoxy or the like. Aryl has the same meaning as the aforementioned aryl moiety of aroyl or aryloxycarbonyl, and the substituents of the substituted aryl have the same meaning as the aforementioned substituents of aroyl or aryloxycarbonyl.

The functional groups of the side chains of amino acid residues constituting B may be chemically modified or protected. Examples of such amino acid residues whose side-chain functional groups are chemically modified or protected are aspartic acid and glutamic acid residues whose side-chain carboxyl group is protected by a benzyl ester, cysteine residue whose side-chain thiol group has been carboxymethylated, or the like.

Examples of pharmaceutically acceptable salts are acid addition salts, metal salts, organic base addition salts, and the like. Examples of acid addition salts include inorganic acid salts such as hydrochloride, sulfate, phosphate, and the like; and organic acid salts such as acetate, maleate, fumarate, tartarate, citrate, and the like. Examples of metal salts include alkali metal salts such as sodium salt, potassium salt, and the like; alkaline earth metal salts such as magnesium salt, calcium salt, and the like, aluminum salt, zinc salt, and the like. Examples of organic base addition salts include salts formed with primary amines such as methyl amine, ethyl amine, aniline, and the like; secondary amines such as dimethyl amine, diethyl amine, pyrrolidine, piperidine, morpholine, piperazine, and the like; and tertiary amines such as trimethylamine, triethylamine, *N,N*-dimethylaniline, pyridine, and the like; ammonium salt, and the like.

Specific examples of the peptides of the present invention include (i) a peptide comprising the amino acid sequence shown in SEQ ID NO: 1, (ii) a peptide comprising the amino acid sequence shown in SEQ ID NO: 2, (iii) a peptide in formula (II), where $R^1$ is a hydrogen atom, B is a peptide residue comprising the amino acid sequence shown in SEQ ID NO: 3, and $R^2$ is an unsubstituted amino, or more specifically, a peptide comprising a sequence with a C-terminal amidated proline residue in the amino acid sequence shown in SEQ ID NO: 3 (the amino acid sequence of SEQ ID NO: 26), (iv) a peptide in formula (II), where $R^1$ is a hydrogen atom, B is a peptide residue comprising the amino acid sequence shown in SEQ ID NO: 4, and $R^2$ is unsubstituted amino, or more specifically, a peptide comprising a sequence with a C-terminal amidated proline residue in the amino acid sequence shown in SEQ ID NO: 4 (the amino acid sequence of SEQ ID NO: 27), (v) a peptide comprising the amino acid shown in SEQ ID NO: 28, (vi) a peptide comprising the amino acid sequence shown in SEQ ID NO: 29, (vii) a peptide comprising the amino acid sequence shown in SEQ ID NO: 6, (viii) a peptide comprising the amino acid sequence shown in SEQ ID NO: 21, and (ix) a peptide comprising the amino acid sequence shown in SEQ ID NO: 22. The peptides of (vii) to (ix) are based on the peptides of (ii) to (iv), respectively, which are sequences of rat VGF.

2. Methods for producing the peptides of the present invention

(1) Chemical synthetic production method

Peptides of the present invention can be obtained by synthesis, followed by purification, using general peptide synthesis methods described in, for example, Izumiya, N., Kato, T., et al., "Fundamentals and Experiments of Peptide Synthesis (Peptide Gosei no Kiso to Jikken)", Maruzen, (1985); Aimoto, S. et al., "Experimental Chemical Course (Jikken Kagaku Koza)", ed. 4, vol. 22, "Organic Synthesis (Yuki Gosei) IV, Acid, Amino acid and Peptide", Maruzen, (1999); Int. J. Pept. Protein Res. 35, 161-214 (1990); Fields, G. B., Solid-Phase Peptide Synthesis, Methods in Enzymology, vol. 289, Academic Press, (1997); Pennington, M. W. and Dunn, B. M., Peptide Synthesis Protocols, Methods in Molecular Biology, vol. 35, Humana Press, (1994), and the like. Specific methods of synthesis include an azide method, acid chloride method, acid anhydride method, mixed acid anhydride method, dichloromethane method, active ester method, carboimidazole method, oxidation-reduction method, and the like. Furthermore, both solid-phase synthesis methods and liquid-phase synthesis methods can be applied to such synthesis. More specifically, a peptide of interest can be synthesized by condensing amino acids constituting a peptide of the present invention with a residual moiety, and by removing protecting groups when the product has a protecting group.

Furthermore, when the side chain of the amino acid residue constituting the peptide, the peptide's amino terminus, and/or the peptide's carboxyl terminus is chemically modified or protected, peptides of the present invention can be produced by methods conventionally known in the field of peptide synthetic chemistry, such as methods of chemical modification after peptide synthesis, methods of peptide synthesis using a chemically modified amino acid, methods of appropriately selecting reaction conditions for the final deprotection in peptide synthesis, or the like (Izumiya, N., et al., "Fundamentals and Experiments of Peptide Synthesis (Peptide Gosei no Kiso to Jikken)", Maruzen, 1985; Yajima, H. ed "The sequel of Development of Pharmaceuticals (Zoku Iyakuhin no Kaihatsu)", vol. 14, Peptide Synthesis, Hirokawa Shoten, 1991; The Japanese Biochemical Society ed., "Biochemistry Experimental Course (Seikagaku Jikken Koza)", vol. 1, "Chemistry of Protein IV-Chemical Modification and Peptide Synthesis", Tokyo Kagaku Dojin; and Ohno, M., et al., "Experimental Methods in Biological Chemistry (Seibutsukagaku Jikkenho)" vols. 12 and 13, "Chemical Modification of Proteins (Tanpakushitsu no Kagaku Shushoku), I and II", Japan Scientific Societies Press, 1981.

In addition, the peptides of the present invention can be synthesized by an automated peptide synthesizer. The synthesis of the peptides by use of a peptide synthesizer is carried out, using amino acids with appropriately protected side chains, such as N $\alpha$-Fmoc (9-fluorenylmethyloxycarbonyl)-amino acids, N $\alpha$-Boc ($t$-butyloxycarbonyl)-amino acids, and the like, on a commercially available peptide synthesizer, for example, a peptide synthesizer manufactured by Shimadzu Corporation, a peptide synthesizer manufactured by Advanced ChemTech Inc., or the like, according to the respective synthesis programs. Protected amino acids and carrier resins used as source materials are available from Applied Biosystems, Shimadzu Corporation, Kokusan Kagaku (Kokusan Chemical Co., Ltd), EMD Biosciences, Inc., Watanabe Kagaku (Watanabe Chemical Industries, Ltd), Advanced Chemtech, Ana Spec, Inc., Peptide Institute, Inc, and the like.

The peptides of the present invention can be purified by combining general purification methods such as solvent extraction, distillation, column chromatography, liquid chromatography, recrystallization, and the like.

(2) Methods of production using genetic engineering techniques

When a peptide of the present invention comprises the aforementioned 20 essential amino acids, and if its side chain, N terminus, or C terminus is not modified, it can be produced by methods described in Molecular Cloning: A Laboratory Manual, 3rd edition, Cold Spring Harbor Laboratory Press (2001), or the like. For example, the peptides can be produced by expressing a DNA encoding a peptide of the present invention in a host cell by the following method.

DNAs encoding the peptides of the present invention can be synthesized using a DNA synthesizer by designing nucleotide sequences coding the amino acid sequences of the peptides of the present invention. When the peptides of the present invention are partial peptides of human VGF comprising the sequence shown in any of SEQ ID NOS: 1 to 4, they can be isolated by PCR using cDNAs of human brain cells or pancreatic cells as template. When DNAs encoding the peptides of the present invention are used to produce the peptides, stop codons are placed at the terminal ends of the regions encoding the peptides. Examples of DNAs encoding the peptides of the present invention comprise the amino acid sequence shown in SEQ ID NO: 1, 2, 3, 4, 28 or 29, include DNAs comprising the nucleotide sequence shown in SEQ ID NOS: 10, 11, 12, 13, 34 or 35, respectively.

A method for producing the peptides of the present invention when the N terminus is a methionine is described below.

A recombinant vector is prepared by inserting a DNA encoding a peptide of the present invention obtained as described above to the downstream of a promoter of a suitable expression vector, and the recombinant vector is introduced into a host cell that is appropriate for the expression vector.

For example, any bacteria, yeasts, animal cells, insect cells, plant cells, and the like can be used as host cells so long as they can express the gene of interest.

Expression vectors that are used are those that can replicate autonomously in the above-mentioned host cells or can be integrated into a chromosome, and which contain a promoter at a position where the DNA encoding the peptide of the present invention can be transcribed.

When prokaryotes, such as bacteria or the like, are used as host cells, it is preferred that the recombinant vectors comprising the DNAs encoding the peptides of the present invention can replicate autonomously in prokaryotes, and at the same time, that the vectors are composed of a promoter, a ribosome-binding sequence, a DNA of the present invention, and a transcription termination sequence. A gene regulating the promoter may also be included.

Examples of the expression vectors are pSE420 (Invitrogen), pGEMEX-1 (Promega), pQE-30 (QIAGEN), pKYP10 (Japanese Published Unexamined Patent Application No. 110600/83), pKYP200 (Agric. Biol. Chem., 48, 669 (1984)), pLSA1 (Agric. Biol. Chem., 53 277 (1989)), pGEL1 (Proc. Natl. Acad. Sci., USA, 82, 4306 (1985)), pBluescript II SK(-) (Stratagene), pTrs30 (prepared from transformed *E. coli* cell line (FERM BP-5407)), pTrs32 (prepared from transformed *E. coli* cell line (FERM BP-5408)), pGHA2 (prepared from transformed *E. coli* cell line (FERM BP-400)), pGKA2 (prepared from transformed *E. coli* cell line (FERM BP-6798)), pTerm2 (Japanese Published Unexamined Patent Application No. 22979/91), pGEX-2T (GE Healthcare), pET (Novagen), pKK223-2 (GE Healthcare), pMAL-c2X (New England Biolabs), and the like.

Any promoter can be used, so long as it can function in the host cells. Examples include promoters derived from *E. coli,* phage, and the like, such as trp promoter ($P_{trp}$), lac promoter, $P_L$ promoter, $P_R$ promoter, T7 promoter, and the like. In addition, artificially designed and modified promoters, such as a promoter in which two $P_{trp}$'s are linked in tandem ($P_{trp}$x2), tac promoter, lacT7 promoter, letI promoter, and the like, can be used.

It is preferred to use a plasmid in which the distances between the Shine-Dalgarno sequence which is ribosome binding sequence, and initiation codon are appropriately adjusted (for example, 6 to 18 bases).

In the recombinant vector of the present invention, a transcription termination sequence is not always necessary for the expression of the DNA of the present invention; however, it is preferred to place the transcription termination sequence immediately downstream of the structural gene.

Examples of host cells include microorganisms belonging to the genera *Escherichia, Serratia, Bacillus, Brevibacterium, Corynebacterium, Microbacterium, Pseudomonas,* and the like, such as *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* TB1, *Serratia ficaria, Serratia fonticola, Serratia liquefaciens, Serratia marcescens, Bacillus subtilis, Bacillus amyloliquefaciens, Brevibacterium ammoniagenes, Brevibacterium immariophilum* ATCC 14068, *Brevibacterium saccharolyticum* ATCC 14066, *Brevibacterium flavum* ATCC 14067, *Brevibacterium lactofermentum* ATCC 13869, *Corynebacterium glutamicum* ATCC 13032, *Corynebacterium glutamicum* ATCC13869, *Corynebacterium acetoacidophilum* ATCC 13870, *Microbacterium ammoniaphilum* ATCC 15354, *Pseudomonas putida, Pseudomonas* sp. D-0110, and the like.

As the method for introducing the recombinant DNAs, any method for introducing a DNA into the above-mentioned host cells can be used, and examples include methods using calcium ion (Proc. Natl. Acad. Sci. USA, 69, 2110 (1972)), protoplast methods (Japanese Published Unexamined Patent Application No. 2483942/88), methods described in Gene, 17, 107 (1982) and Molecular & General Genetics, 168, 111 (1979), and the like.

When yeasts are used as host cells, the expression vector may be, for example, YEp13 (ATCC37115), YEp24 (ATCC37051), YCp50 (ATCC37419), pHS19, pHS15, or the like.

Any promoter can be used, so long as it can be expressed in a yeast cell line; and examples include promoters of genes in the glycolytic pathway such as hexose kinase and the like, PHO5 promoter, PGK promoter, GAP promoter, ADH promoter, gal 1 promoter, gal 10 promoter, a heat shock polypeptide promoter, MF α1 promoter, CUP 1 promoter, and the like.

The host cells include microorganisms belonging to the genera *Saccharomyces, Schizosaccharomyces, Kluyveromyces, Trichosporon, Schwanniomyces, Pichia, Candida* and the like, and examples include *Saccharomyces cerevisiae, Schizosaccharomyces pombe, Kluyveromyces lactis, Trichosporon pullulans, Schwanniomyces alluvius, Candida utilis,* and the like.

Any method for introducing a recombinant vector into yeast host cells can be used, so long as it ensures the introduction of DNA into yeast. Such methods include, for example, electroporation (Methods. in Enzymol., 194, 182 (1990)), the spheroplast method (Proc. Natl. Acad. Sci. USA, 75, 1929 (1978)), the lithium acetate method (J. Bacteriology., 153, 163 (1983)), and the method described in Proc. Natl. Acad. Sci. USA, 75, 1929 (1978).

When an animal cell is used as the host cell, suitable expression vectors include, for example, pcDNA3.1(+) (Invitrogen), pAGE107 (Japanese Published Unexamined Patent Application No. 22979/91; Cytotechnology, 3, 133 (1990)), pAS3-3 (Japanese Published Unexamined Patent Application No. 227075/90), pCDM8 (Nature, 329, 840 (1987)), pREP4 (Invitrogen), pAGE103 (J. Biochem., 101, 1307 (1987)) and the like.

Any promoter can be used, so long as it functions in the animal cells. Such promoters include, for example, the promoter of the IE (immediate early) gene of cytomegalovirus (CMV), SV 40 early promoter, retroviral promoter, metallothionein promoter, heat-shock promoter, and SRα promoter. Further, the enhancer of the IE gene of human CMV may be used in combination with a promoter.

The host cells to be used in the present invention include Namalwa cell, a human cell line; COS cell, derived from monkey; CHO cell, derived from Chinese hamster; HBT5637 (Japanese Published Unexamined Patent Application No. 299/88) and the like.

Any of the methods for introducing a recombinant vector into animal host cells can be used as long as it ensures the introduction of a DNA into animal cells. Such methods include, for example, electroporation (Cytotechnology, 3, 133 (1990)), the calcium phosphate method (Japanese Published Unexamined Patent Application No. 227075/90), and the lipofection method (Proc. Natl. Acad. Sci. USA, 84, 7413 (1987); Virology, 52, 456 (1973)).

When insect cells are used as host cells, a peptide can be expressed, for example, by the method described in Current Protocols in Molecular Biology; Baculovirus Expression Vectors, A Laboratory Manual, W.H. Freeman and Company, New York (1992); Bio/Technology, 6, 47 (1988), or the like.

More specifically, a vector for introducing a recombinant gene and a genome deficient baculovirus are co-transfected into insect cells to obtain a recombinant virus in the supernatant of an insect cell culture, and then the insect cells are infected with the recombinant virus to express the peptides.

Gene introducing vectors used in this method include, for example, pVL1392, pVL1393 (Becton, Dickinson and Company), pBlueBac4.5 (Invitrogen), and the like.

Baculoviruses that can be used in the present invention include, for example, *Autographa californica,* a nuclear polyhedrosis virus that is infectious to insects belonging to the family of Cabbage armyworm.

Insect cell that can be used in the present invention include, for example, Sf9 and Sf21 both of which are ovarian cells of *Spodoptera frugiperda* (Baculovirus Expression Vectors, A Laboratory Manual, W. H. Freeman and Company, New York, (1992)); and High5 (Invitrogen) that is an ovarian cell of *Trichoplusia ni;* and the like.

Methods for co-introducing the above-mentioned recombinant gene introducing vector and the baculovirus into insect cells to prepare recombinant viruses include, for example, the calcium phosphate method (Japanese Published Unexamined Patent Application No. 227075/90) and the lipofection method (Proc. Natl. Acad. Sci. USA, 84, 7413 (1987)).

When plant cells are used as host cells, the expression vector includes, for example, Ti plasmid, the tobacco mosaic virus vector, and the like.

Any promoter can be used, so long as it can be expressed in a plant cell, and examples include the 35S promoter of cauliflower mosaic virus, rice actin 1 promoter, and the like.

The host cells include such as plant cells and the like of tobacco, potato, tomato, carrot, soybean, rapeseed, alfalfa, rice, wheat, barley, and the like.

Any method for introducing the recombinant vector can be used, so long as it is a method for introducing a DNA into plant cells, and examples include the *Agrobacterium* method (Japanese Published Unexamined Patent Application No. 140885/84, Japanese Published Unexamined Patent Application No. 70080/85, WO 94/00977), electroporation method (Japanese Published Unexamined Patent Application No. 251887/85), particle gun method (Granted/Registered Japanese Patents 2606856 and 2517813), and the like.

A peptide of the present invention can be produced by culturing a transformant of the present invention which is obtained as described above in a medium to produce and accumulate the peptide of the present invention in the culture, and recovering it from the same.

The method for culturing the transformant of the present invention in a medium can be carried out according to the general methods that are used in culturing the host.

When a transformant of the present invention is obtained by using a prokaryote such as *Escherichia coli* or an eukaryote such as yeast as a host, the medium used for culturing may be either a natural medium or a synthetic medium, so long as it contains a carbon source, a nitrogen source, inorganic salts, and the like, and these can be assimilated by the transformant so that the transformant can be cultured efficiently.

Any carbon source can be used, so long as it can be assimilated by the transformant, and examples include carbohydrates such as glucose, fructose, sucrose, molasses containing them, starch, starch hydrolysate, and the like; organic acids such as acetic acid, propionic acid, and the like; alcohols such as ethanol, propanol, and the like.

The nitrogen source includes ammonia, ammonium salts of inorganic acids or organic acids such as ammonium chloride, ammonium sulfate, ammonium acetate, ammonium phosphate, and the like, other nitrogen-containing compounds, peptone, meat extract, yeast extract, corn steep liquor, casein hydrolysate, soybean meal and soybean meal hydrolysate, various fermenting microbial cells and the digest thereof, and the like.

The inorganic salts that can be used are, for example, monopotassium phosphate, dipotassium phosphate, magnesium phosphate, magnesium sulfate, sodium chloride, ferrous sulfate, manganese sulfate, copper sulfate, calcium carbonate, and the like.

Culturing is preferably carried out under aerobic conditions by shaking culture, submerged spinner culture under aeration, or the like. The culturing temperature is preferably 15°C to 40°C, and the preferred culturing time is generally 16 hours to 7 days. The pH is preferably maintained at 3.0 to 9.0 during culturing. The pH can be adjusted by using an inorganic or organic acid, an alkali solution, urea, calcium carbonate, ammonia, or the like.

Also, antibiotics such as ampicillin, tetracycline, and the like can be added to the medium during culturing, if necessary.

When culturing a microorganism transformed with a recombinant vector that uses an inducible promoter as a promoter, an inducer can be added to the medium, if necessary. For example, isopropyl-β-D-thiogalactopyranoside or the like can be added to the medium when culturing a microorganism transformed with a recombinant vector having a lac promoter, or indoleacrylic acid or the like can be added to the medium when culturing a microorganism transformed with a recombinant vector having a *trp* promoter.

The medium for culturing a transformant obtained using animal cells as host includes RPMI 1640 medium (The Journal of the American Medical Association, 199, 519 (1967)), Eagle's minimum essential medium (MEM) (Science, 122, 501 (1952)), modified Dulbecco's Eagle medium (Virology, 8, 396 (1959)), 199 Medium (Proceeding of the Society for the Biological Medicine, 73, 1 (1950)), and the above media with added fetal calf serum or the like.

Generally, culturing is preferably carried out in the presence of 5% $CO_2$ at pH 6 to 8 and at a temperature of 30°C to 40°C for one to seven days.

Furthermore, antibiotics such as kanamycin, penicillin, and the like can be added to the medium while culturing, if necessary.

The level of production can be increased using a gene amplification system that uses a dihydrofolate reductase gene, or the like according to the method described in the Japanese Published Unexamined Patent Application No. 227075/90.

The medium used for culturing a transformant obtained using insect cells as host includes generally used medium, such as TNM-FH medium (Becton Dickinson), Sf-900 II SFM medium (Invitrogen), ExCell 400 and ExCell 405 (both manufactured by JRH Biosciences), Grace's Insect Medium (Nature, 195, 788 (1962)), or the like.

Generally, culturing is preferably carried out at pH 6 to 7 and at a temperature of 25°C to 30°C for one to five days.

Furthermore, antibiotics such as gentamicin and the like may be added to the medium while culturing, if necessary.

A transformant obtained using plant cells as host can be cultured as cells or cultured after having differentiated into plant cells or organs. The medium used for culturing the transformant includes generally used medium, such as Murashige and Skoog medium, White medium, and the above media with added plant hormones such as auxin, cytokinine, or the like.

Generally, culturing is preferably carried out at pH 5 to 9 and at a temperature of 20°C to 40°C for three to 60 days.

Furthermore, antibiotics such as kanamycin, hygromycin, and the like can be added to the medium while culturing, if necessary.

As described above, the peptides of the present invention can be produced by culturing a transformant derived from a microorganism, an animal cell, or a plant cell containing a recombinant vector into which a DNA encoding the peptide of the present invention has been incorporated, to form and accumulate the peptide according to general culturing methods, and by collecting the peptide from culture.

The peptides of the present invention can be produced by preparing a fusion protein between any polypeptide (hereinafter referred to as polypeptide X) and a peptide of the present invention, and then isolating the peptide of the present invention from the fusion protein so as to avoid being degraded in the host cell. An expression vector that expresses the fusion protein can be prepared by adding a DNA encoding methionine or a specific protease recognition sequence to the 5'-end of the above-mentioned DNA encoding the peptide of the present invention, and then ligating it in frame with a DNA encoding polypeptide X in a polypeptide X expression vector. However, a methionine-encoding DNA is added only when the peptide of the present invention does not contain methionine. Any polypeptide may be used as polypeptide X, and examples include glutathione S-transferase, maltose binding protein, DsbA, DsbC, protein A, and the like. Examples of a specific protease recognition sequence are factor Xa recognition sequence (Ile-Glu-Gly-Arg), enterokinase recognition sequence (Asp-Asp-Asp-Asp-Lys), and the like. The polypeptide X expression vector can be prepared similarly to the above-mentioned expression vector for the peptide of the present invention, by inserting a DNA encoding polypeptide X instead of a DNA encoding the peptide of the present invention. Commercially available vectors for expressing a fusion protein, for example pGEX-3 vector for expressing a fusion protein with glutathione S-transferase (GE Healthcare), pMAL-c2X and pMAL-p2E vectors for expressing a fusion protein with a maltose binding protein (New England BioLabs), pET-39b(+) vector for expressing a fusion protein with DsbA (EMD Biosciences), or the like can also be used. If the peptide of the present invention is fused to polypeptide X via a specific protease recognition sequence, the peptide of the present invention can be cleaved from the fusion protein by treatment with a corresponding protease for the recognition sequence. If the peptide of the present invention is fused to the C terminus of polypeptide X via methionine, the peptide of the present invention can be cleaved from the fusion protein by cyanogen bromide treatment according to the method described in Japanese Published Unexamined Patent Application No. 102096/89. Subsequent to the treatment with protease or cyanogen bromide, the peptide of the present invention can be isolated and purified by combining gel filtration, reverse-phase HPLC, affinity chromatography, and the like.

The peptide of the present invention can be produced by adding a DNA encoding the signal peptide of a secretory protein to the 5' end of a DNA encoding the peptide of the present invention, using this DNA to prepare a recombinant vector in the same manner as described above, and transfecting a host cell with the vector and allowing secretion of the polypeptide into the medium as described in the following literature (J. Biol. Chem., 264, 17619 (1989); Proc. Natl. Acad. Sci., USA, 86, 8227 (1989); Genes Develop., 4, 1288 (1990); Japanese Published Unexamined Patent Application Nos. 336963/93; WO94/23021).

If the N terminus of the peptide of the present invention is not a methionine, the peptide can be produced by a method of producing the above-mentioned fusion protein and isolating or secreting the peptide into medium. For example, the peptide of the present invention comprising the amino acid sequence shown in SEQ ID NO: 2 is prepared as follows. First, a DNA comprising the nucleotide sequence shown in SEQ ID NO: 11 and a DNA comprising a nucleotide sequence that is complementary to the sequence of SEQ ID NO: 11 are chemically synthesized in a DNA synthesizer, and then the two are annealed to prepare a double-stranded DNA. The double-stranded DNA and an XmnI-cleaved pMAL-c2X are ligated to produce a plasmid in which the double-stranded DNA is inserted into the XmnI site of pMAL-c2X. The obtained plasmid encodes a fusion protein, in which a peptide comprising a factor Xa recognition sequence (Ile-Glu-Gly-Arg) and the amino acid sequence shown in SEQ ID NO: 2 is fused at the C terminus of the maltose binding protein. *Escherichia coli* is transformed using the obtained plasmid. The obtained transformant is cultured in a medium, and the fusion protein is expressed in the transformed cells. The cultured bacterial cells are isolated by centrifugation and disrupted, and a solution containing the fusion protein is obtained. The fusion protein is isolated from the obtained solution by affinity chromatography using a maltose-immobilized column, and then the fusion protein is treated with factor Xa to excise the peptide comprising the amino acid sequence shown in SEQ ID NO: 2 from the fusion protein. The peptide comprising the amino acid sequence shown in SEQ ID NO: 2 can be isolated and purified by gel filtration, reverse phase HPLC, or the like.

General methods for isolating and purifying proteins can be used to isolate and purify peptides produced from transformants of the present invention.

For example, when the peptide of the present invention is expressed in a soluble form in cells, the cells are collected by centrifugation upon completion of culturing, suspended in an aqueous buffer, and disrupted using an ultrasonicator, a French press, a Manton Gaulin homogenizer, a Dynomill, or the like to obtain a cell-free extract. A purified product can be obtained from the supernatant obtained by centrifuging the cell-free extract by general methods used for isolating and purifying a protein. More specifically, such methods can be used alone or in combination, and include solvent extraction, salting-out using ammonium sulfate or the like, desalting, precipitation using an organic solvent, anion exchange chromatography using resin, such as diethylaminoethyl (DEAE)-Sepharose, DIAION HPA-75 (Mitsubishi Chemical), or the like, cation exchange chromatography using resin, such as S-Sepharose FF (Pharmacia) or the like, hydrophobic chromatography using resin, such as butyl sepharose, phenyl sepharose, or the like, gel filtration using a molecular sieve, affinity chromatography, chromatofocusing, and electrophoresis such as isoelectronic focusing or the like.

If the peptide is expressed in an insoluble form in cells, the cells are collected in the same manner, and then disrupted and centrifuged to recover the insoluble form of the peptide as a precipitated fraction. The collected insoluble form of the peptide is solubilized with a protein denaturing agent. The solubilized solution is diluted or dialyzed to reconstitute the normal tertiary structure of the peptide by lowering the concentration of the protein-denaturing agent in the solubilized solution. Subsequent to this procedure, a purified product of the peptide can be obtained by the same purification and isolation method described above.

If the peptide of the present invention is secreted extracellularly, the peptide can be collected in the culture supernatant. Specifically, the culture supernatant is obtained by treating the culture in the same method described above, such as centrifugation or the like, and a purified product can be obtained from the culture supernatant using the same purification and isolation method described above.

3. Antibodies that specifically bind to the peptides of the present invention

Antibodies of the present invention bind to an epitope present in the amino acid sequence shown in SEQ ID NO: 4 or in a sequence in which the C terminus of the amino acid sequence shown in SEQ ID NO: 4 is amidated (SEQ ID NO: 27), and can bind specifically to peptides of the present invention described in 1. The antibodies of the present invention may be polyclonal antibodies or monoclonal antibodies. Antibodies of the present invention include antibody fragments such as Fab, Fab', F(ab')$_2$ prepared from polyclonal antibodies or monoclonal antibodies. The monoclonal antibodies include humanized chimeric antibodies comprising a constant region of a human antibody and a variable region of a monoclonal antibody produced in a non-human animal, and humanized CDR-grafted antibodies comprising a human antibody constant region and a variable region with complementarity-determining regions (CDRs) of a monoclonal antibody produced in a non-human animal inserted into a human framework region.

(1) Production of polyclonal antibodies

Polyclonal antibodies that bind to an epitope present in the amino acid sequence shown in SEQ ID NO: 4 or in a sequence in which the C terminus of the amino acid sequence shown in SEQ ID NO: 4 is amidated (SEQ ID NO: 27), can be prepared as follows. A peptide antigen comprising the amino acid sequence shown in SEQ ID NO: 4 or a sequence in which the C terminal proline residue in the amino acid sequence shown in SEQ ID NO: 4 is amidated (SEQ ID NO: 27), is intradermally, intravenously, intraperitoneally or intramuscularly administered to a non-human animal. These polyclonal antibodies can bind specifically to the peptides of the present invention. In this case, it is desirable to covalently bind the antigenic peptide to a carrier protein such as keyhole limpet hemocyanin, bovine thyroglobulin, ovalbumin, or the like, and administer it with an adjuvant. The antigenic peptide can be covalently bound to a carrier protein by performing reactions using cross-linking reagents such as maleimide, carbodiimide, glutaraldehyde, and the like. In the case of a maleimide reaction, a peptide in which a cysteine residue has been added to the N terminus or C terminus of the amino acid sequence of the antigenic peptide is prepared by the method described in 2, and covalently bound via cysteine. Examples of an adjuvant include Freund's complete adjuvant, aluminum hydroxide gel, pertussis vaccine, and the like. A rabbit, goat, rat, mouse, hamster, or the like can be used as a non-human animal to be administered with the antigen, and the dose per administration for each animal is preferably an amount that contains 50 to 200 $\mu$g of the antigenic peptide.

The antigen is preferably administered, for example, every one to three weeks for three to ten times after the first administration until the antibody titer of the serum has sufficiently increased. Serum antibody titer can be measured by preparing serum samples from blood collected three to seven days after each administration, and using an enzyme immunoassay method, radioimmunoassay method, or the like. With reference to Enzyme-linked Immunosorbent Assay (ELISA), Igaku Shoin (1976) and Antibodies - A Laboratory Manual, Cold Spring Harbor Laboratory (1988), the enzyme immunoassay method can be performed based on the procedure of: (i) covalently binding an antigenic peptide to a carrier protein that is different from the one used for the antigen, and immobilizing it onto an appropriate plate, (ii) blocking and washing the plate, (iii) reacting the plate with the serum prepared from the immunized animal and then washing it, (iv) reacting with an enzyme-labeled antibody against IgG of the immunized animal and then washing it, and then (v) reacting the plate with a substrate that develops color or luminesces from the label enzyme and measuring the level of coloring or luminescence as an indicator of antibody titer.

Serum is prepared by collecting blood from a non-human animal that shows a sufficient antibody titer against the antigenic peptide in its serum. This serum, or specifically antiserum, can be used as a polyclonal antibody; alternatively, a polyclonal antibody can be purified from this antiserum.

The method for purifying a polyclonal antibody from antiserum includes, for example, centrifugation; salting out with 40-50% saturated ammonium sulfate; caprylic acid precipitation (Antibodies, A Laboratory manual, Cold Spring Harbor Laboratory (1988)); and chromatography using a DEAE-sepharose column, an anion exchange column, a protein A- or G-column, a gel filtration column, and the like, which may be carried out alone or in combination.

(2) Production of monoclonal antibodies

Monoclonal antibodies that bind to an epitope present in the amino acid sequence shown in SEQ ID NO: 4 or in a sequence in which the C terminus of the amino acid sequence shown in SEQ ID NO: 4 is amidated (SEQ ID NO: 27) can be prepared by the following methods. These monoclonal antibodies can bind specifically to peptides of the present invention.

(a) Preparation of antibody-producing cells

Mice and rats are used as animals for antigen administration. The same antigen used for the production of polyclonal antibodies of (1) is administered, and a mouse or rat that shows a sufficient antibody titer against the antigenic peptide in its serum can be used as a supply source of antibody-producing cells. Splenocytes can be used as antibody-producing cells. The antibody-producing cells can be prepared from a mouse or rat that shows a sufficient antibody titer, for example, as described below.

The spleen of the mouse or rat which showed the antibody titer is excised three to seven days after the final administration of the antigen. The spleen is cut into pieces in MEM, the cells are loosened using a pair of forceps and centrifuged, the supernatant is discarded, and the precipitated splenocytes are collected. The obtained splenocytes are treated with Tris-ammonium chloride buffer (pH 7.65) for one to two minutes to remove erythrocytes and then washed three times with MEM, and the resulting splenocytes are used as antibody-producing cells.

(b) Preparation of myeloma cells

Cells of a cell line established from mouse or rat myeloma cells can be used as myeloma cells. Examples of myeloma cell lines include 8-azaguanine-resistant mouse (BALB/c-derived) myeloma cell lines P3-X63Ag8-U1 (Curr. Topics. Microbiol. Immunol., 81, 1 (1978); Europ. J. Immunol., 6, 511 (1976)), SP2/0-Agl4 (Nature, 276, 269 (1978)), P3-X63-Ag8653 (J. Immunol., 123, 1548 (1979)), P3-X63-Ag8 (Nature, 256, 495 (1975)), and the like. These cell lines are preferably subcultured in 8-azaguanine medium (a medium produced by supplementing RPMI-1640 medium with glutamine (1.5 mmol/L), 2-mercaptoethanol (5 x $10^{-5}$ mol/L), gentamicin (10 $\mu$g/ml) and fetal calf serum (10%) (hereinafter referred to as "normal medium"), and further supplemented with 8-azaguanine (15 $\mu$g/ml)); and it is preferable to culture in the normal medium for three to four days before cell fusion. Preferably, 2 x $10^{7}$ or more cells

are used in fusion.

(c) Production of hybridomas

Hybridomas can be produced by fusing the antibody-producing cells obtained in (a) with the myeloma cells obtained in (b), for example, by using polyethylene glycol as follows. The antibody-producing cells obtained in (a) and the myeloma cells obtained in (b) are washed well with MEM or PBS (1.83 g of disodium phosphate, 0.21 g of monopotassium phosphate, 7.65 g of sodium chloride and one liter of distilled water, pH 7.2), mixed in a ratio of 5:1 1 to 10:1 (antibody-producing cell:myeloma cell), and centrifuged at 1,200 rpm for five minutes, and then the supernatant is discarded. Cells of the obtained precipitation fraction are thoroughly loosened. 2 g of polyethylene glycol-1000, 2 mL of MEM, and 0.7 ml of dimethyl sulfoxide are mixed, and 0.2 to 1 mL of the prepared solution is added for every $10^8$ antibody-producing cells while stirring at 37°C, and then 1 to 2 mL of MEM is added several times every one to two minutes. After the addition, the total volume is adjusted to 50 mL by adding MEM. The prepared solution is centrifuged at 900 rpm for five minutes, and then the supernatant is discarded.

For example, the fused cells can be cultured as described below, and hybridomas with high levels of antibody production can be selected. Cells obtained in the precipitation fraction are loosened gently and then suspended in 100 mL of HAT medium (a medium produced by supplementing the normal medium with hypoxanthine ($10^{-4}$ mol/L), thymidine (1.5 x $10^{-5}$ mol/L), and aminopterin (4 x $10^{-7}$ mol/L)), by repeated gentle sucking and squirting with a measuring pipette. The suspension is preferably dispensed into a 96-well incubation plate at 100 $\mu$L per well and cultured in a 5% $CO_2$ incubator at 37°C for 7 to 14 days. After culturing, a portion of the culture supernatant is collected, and is used instead of serum for measuring the antibody titer as described above in (1). Hybridomas with culture supernatants that have high antibody titer can be selected as hybridomas with high-level antibody production. The hybridoma can be cloned, from which clones with high antibody productivity can be selected to obtain hybridoma cells that steadily show high levels of antibody production. Cloning can be performed, for example, by limiting dilution or the like, and is preferably repeated twice by using HT medium (a medium in which aminopterin is removed from HAT medium) for the first cloning and the normal medium for the second cloning. The above-mentioned antibody titer measurement is performed using the culture supernatant of each of the clones obtained by cloning, and a hybridoma clone whose culture supernatant has high antibody titer can be selected as a hybridoma cell that steadily shows high antibody production.

(d) Preparation of monoclonal antibodies

Monoclonal antibodies of the present invention can be prepared, for example, as described below from ascites where hybridomas selected in (c) are allowed to proliferate as ascites carcinoma in nude mice. Preferably, the hybridoma cells obtained in (c), which produce monoclonal antibodies of the present invention, are administered by intraperitoneal injection at a dose of 5 to 20 x $10^6$ cells/animal to 8- to 10-weeks-old mice or nude mice that have been administered with 0.5 mL of 2,6,10,14-tetramethylpentadecane (pristane) intraperitoneally and reared for 2 weeks. Ten to 21 days later, ascitic fluid is collected from the mouse in which the hybridoma has caused ascites tumor, and this is centrifuged at 3,000 rpm for 5 minutes to remove solid matter. The monoclonal antibody can be purified and obtained from the obtained ascites supernatant using the same method for polyclonal antibody.

The subclass of the antibody can be determined using a mouse monoclonal antibody typing kit or a rat monoclonal antibody typing kit. The amount of peptide can be determined by the Lowry method or by absorbance at 280 nm.

(3) Methods for measuring the peptides of the present invention using antibodies of the present invention

The peptides of the present invention can be immunologically detected or quantified using the antibodies of the present invention. Examples of immunological detection or quantification methods are competition method, sandwich method, immunohistochemistry, Western blotting, aggregation method ("Tan-Clone-Kotai-Manual (Experimental Manual for Monoclonal Antibody" Kodansha-Scientific, 1987; and "Zoku-Seikagaku Jikken Kouza 5, Meneki-seikagaku Kenkyuho (Sequel to the Lectures on Biochemical Experiments 5, Immunobiochemical research methods)", Tokyo Kagaku Dojin, 1986), and the like.

The competition method includes the following steps: reacting an antibody of the present invention with a test solution and a fixed amount of competing substance; labeling a peptide of the present invention to be measured with an enzyme, biotin, radioisotope, fluorescent substance, or the like; allowing the peptide of the present invention in the test solution and the competing substance to competitively bind the antibody; measuring the amount of competing substance bound to the antibody using the label, and quantifying the peptide of the present invention from the level of binding. Examples include a method of fixing the antibody onto a solid phase such as plates, beads, or the like, allowing the peptide of the present invention and the competing substance to competitively bind the antibody, washing the solid phase, and measuring the amount of competing substance bound to the antibody on the solid phase; a method of allowing the peptide of the present invention and the competing substance to competitively bind the antibody, using $\gamma$-globulin and polyethylene glycol to precipitate immune complexes for separating unbound competing substance, and then measuring the amount of competing substance bound to the antibody; and the like. The peptide of the present invention in the sample solution can be quantified, for example, by preparing five to ten predetermined concentrations of the solution of the peptide of the present invention, measuring the level of binding between the competing substance and the antibody when these solutions are used as a sample solution, producing a standard curve by plotting the peptide concentration versus the binding level of the competing substance. The standard curve can be applied to the binding level of the competing substance to quantify the peptide of the present invention in the test solution.

The sandwich method uses two types of antibodies that bind specifically to a peptide of the present invention. Examples include a method fixing one of the antibodies onto a solid phase such as plates, beads, or the like, reacting a sample solution with this solid phase, and after binding the peptide of the present invention in the sample to the antibody on the solid phase, reacting it with the other antibody which is labeled with an enzyme, biotin, radioisotope, fluorescent substance, or the like, to bind the labeled antibody to the peptide of the present invention bound to the antibody on the solid phase, the binding level of the labeled antibody is determined using the labeling substance, and this binding level is used to quantify the peptide of the present invention. The peptide of the present invention in the sample solution can be quantified, for example, by preparing five to ten fixed predetermined concentrations of the solution of the peptide of the present invention, measuring the level of binding the labeled antibody when these solutions are used as a sample solution, producing a standard curve by plotting the peptide concentration versus the binding level of the label. The standard curve can be applied to the binding level of the labeled antibody to quantify the peptide of the present invention in the test solution.

Enzyme immunoassay is a quantification method used to label the competing substance or the antibody in the above-mentioned competition method or sandwich method with an enzyme such as alkaline phosphatase, peroxidase, or the like, react it with a reagent that develops color or luminescence from the label enzyme, and determine the binding level of the competing substance or labeled antibody from the level of color development or luminescence. Furthermore, radioimmunoassay is a quantification method used to label the competing substance or the antibody in the above-mentioned competition method or sandwich method with a radioisotope, and determining the binding level of the competing substance or the labeled antibody from radioactivity.

Immunohistochemistry is used to detect a peptide of the present invention in tissues or cells by reacting a frozen or paraffin-embedded section of tissues or cells with an antibody of the present invention labeled with an enzyme, biotin, radioisotope, fluorescent substance, gold colloid, or the like, and then detecting the antibody of the present invention using the labeling substance.

Western blotting is a method used to separate proteins and peptides included in a sample on an SDS-polyacrylamide gel, blot proteins and peptides from the gel onto a polyvinylidene difluoride (PVDF) membrane, nitrocellulose membrane, or the like, and after reacting this with an antibody of the present invention labeled with an enzyme, biotin, radioisotope, or the like, detect the antibody of the present invention using the labeling substance, and detect the peptide of the present invention on the membrane.

The aggregation method uses absorbance measurement to detect or quantify aggregates of particles formed from reaction of a test solution with latex particles or the like immobilized with an antibody of the present invention, and binding of the antibody on the particles to the peptide of the present invention in the sample.

4. C-terminal VGF-derived peptide-containing pharmaceutical formulations

In the present invention, any of the peptides of (a) to (f) described below is referred to as a "C-terminal VGF-derived peptide". The peptides of the present invention described in 1 are included in the C-terminal VGF-derived peptides:

(a) a peptide comprising the amino acid sequence shown in any of SEQ ID NOS: 1 to 5, 28 and 29 (but excluding peptides comprising the amino acid sequence shown in SEQ ID NO: 9, 30, 31, 32 or 33);
(b) a peptide comprising an amino acid sequence with substitution, deletion, or addition of one to five amino acids in the amino acid sequence shown in any one of SEQ ID NOS: 1 to 5, 28 and 29, wherein the peptide has an activity of increasing intracellular calcium ion concentration of cardiac or vascular cells;
(c) a peptide comprising an amino acid sequence having 90% or higher homology to the amino acid sequence shown in any one of SEQ ID NOS: 1 to 5, 28 and 29, wherein the peptide has an activity of increasing intracellular calcium ion concentration of cardiac or vascular cells;
(d) a peptide represented by formula (I) of 1.;
(e) a peptide comprising the amino acid sequence shown in SEQ ID NO: 23, or a peptide comprising an amino acid sequence that has the amino acid sequence shown in SEQ ID NO: 23 and any sequence comprising one to 32 amino acids added to the N terminus thereof; and
(f) a peptide represented by formula (III) below

$$R^3\text{-}C\text{-}R^4 \qquad (III)$$

(wherein, $R^3$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^4$ represents hydroxy, substituted or unsubstituted alkoxy, or substituted or unsubstituted amino; and C represents a peptide residue of any one of the above-mentioned (a) to (e)).

Amino acid substitution, deletion, and addition, and homology of the amino acid sequence in the "C-terminal VGF-derived peptide" mentioned above have the same definitions as amino acid substitution, deletion, and addition, and homology of the amino acid sequence in the peptide of the present invention in 1. Each group in formula (III) has the same definition as in formula (II) of 1.

The amino acid sequence shown in SEQ ID NO: 23 is the amino acid sequence represented by formula (VIII) below.

Ala-Gln-Glu-Glu-Ala-$X^{13}$-Ala-Glu-Glu- Arg-Arg-Leu-Gln-Glu-Gln-Glu-Glu-Leu-Glu-Asn-Tyr-Ile-Glu-His-Val-Leu-Leu-$X^{14}$-Arg-Pro  (VIII)

(wherein, $X^{13}$ and $X^{14}$ may be the same or different and independently represent an amino acid)

In formula (VIII), preferably $X^{13}$ is aspartic acid, glutamic acid, isoaspartic acid, isoglutamic acid, 2-aminoadipic acid, or 2-aminosuberic acid, and $X^{14}$ is arginine, lysine, ornithine, 2,4-diaminobutanoic acid, 2,3-diaminopropionic acid, or histidine; more preferably $X^{13}$ is aspartic acid or glutamic acid and $X^{14}$ is arginine or histidine; and $X^{13}$ and $X^{14}$ are, respectively, glutamic acid and arginine, or aspartic acid and histidine.

The amino acid sequence comprising the amino acid sequence of SEQ ID NO: 23 and an additional one to 32 amino acids at the N terminus may be any sequence, but preferably the amino acid sequence is represented by formula (IX) (SEQ ID NO: 24) below. Thr-Leu-Gln-Pro-Pro-$X^{15}$-$X^{16}$-$X^{17}$-Arg-Arg-Arg-His-$X^{18}$-His-His-Ala-Leu-Pro-Pro-$X^{19}$-Arg-His

-$X^{20}$-Pro-$X^{21}$-$X^{22}$-Glu-Ala-Gln-Ala-Arg-Arg  (IX)

(wherein, $X^{15}$, $X^{16}$, $X^{17}$, $X^{18}$, $X^{19}$, $X^{20}$, $X^{21}$ and $X^{22}$ may be the same or different and independently represent an amino acid)

In formula (IX), preferably $X^{15}$, $X^{16}$, and $X^{17}$ are serine, threonine, homoserine, leucine, isoleucine, norleucine, valine, norvaline, alanine, 2-aminobutanoic acid, methionine, *O*-methylserine, *t*-butylglycine, *t*-butylalanine, cyclohexylalanine or *tert*-leucine, $X^{18}$ is-tyrosine or phenylalanine, $X^{19}$ is serine, threonine, homoserine, leucine, isoleucine, norleucine, valine, norvaline, alanine, 2-aminobutanoic acid, methionine, *O*-methylserine, t-butylglycine, t-butylalanine, cyclohexylalanine or *tert*-leucine, $X^{20}$ is tyrosine, phenylalanine or histidine, $X^{21}$ is glycine, aspartic acid, glutamic acid, isoaspartic acid, isoglutamic acid, 2-aminoadipic acid or 2-aminosuberic acid, and $X^{22}$ is arginine, lysine, ornithine, 2,4-diaminobutanoic acid, 2,3-diaminopropionic acid, leucine, isoleucine, norleucine, valine, norvaline, alanine, 2-aminobutanoic acid, methionine, *O*-methylserine, t-butylglycine, *t*-butylalanine, cyclohexylalanine or *tert*-leucine; more preferably $X^{15}$ and $X^{16}$ are serine or alanine, $X^{17}$ is serine or leucine, $X^{18}$ is tyrosine or phenylalanine, $X^{19}$ is serine or alanine, $X^{20}$ is tyrosine or histidine, $X^{21}$ is glycine or aspartic acid, and $X^{22}$ is arginine or leucine; and even more preferably $X^{15}$, $X^{16}$, $X^{17}$, $X^{18}$, $X^{19}$, $X^{20}$, $X^{21}$ and $X^{22}$ are, respectively, serine, alanine, leucine, tyrosine, serine, tyrosine, glycine and arginine; or alanine, serine, serine, phenylalanine, alanine, histidine, aspartic acid and leucine.

In addition to the peptides of (i) to (ix) described in 1 as specific examples of the peptides of the present invention, specific examples of the C-terminal VGF-derived peptides include (x) a peptide comprising the amino acid sequence shown in SEQ ID NO: 5, (xi) a peptide comprising the amino acid sequence shown in SEQ ID NO: 25, and (xii) a peptide comprising the amino acid sequence shown in SEQ ID NO: 16. The peptide of (x) is a peptide corresponding to the sequence of positions 586 to 615 at the C terminus of human VGF, the peptide of (xi) is a peptide derived from the rat VGF corresponding to the peptide of (x), and the peptide of (xii) is a peptide derived from the rat VGF corresponding to the peptide of (i) of the present invention.

Since the C-terminal VGF-derived peptides and the pharmaceutically acceptable salts thereof have an activity of increasing intracellular calcium ion concentration of vascular or cardiac cells, they have circulation-modulating activity which modulates blood pressure and the amount of blood flow. Therefore, the C-terminal VGF-derived peptides and the pharmaceutically acceptable salts thereof can be used as active ingredients of circulation-modulating agents, vasopressors, and therapeutic agents for diseases of the circulatory system such as myocardial infarction, ischemic heart disease, cerebral infarction, or the like. Examples of the pharmaceutically acceptable salts of the C-terminal VGF-derived peptides include the pharmaceutically acceptable salts of the peptides of the present invention described in 1.

In pharmaceutical formulations containing a C-terminal VGF-derived peptide or a pharmaceutically acceptable salt thereof, the peptide or the pharmaceutically acceptable salt thereof may be included as an active ingredient as such, or in a mixture with any other therapeutic active ingredient. Such pharmaceutical formulations are produced by any method well known in the technical field of pharmaceutical formulation by mixing the active ingredient with one or more pharmaceutically acceptable carriers.

It is desirable to use the most effective route of administration for carrying out the treatment, and examples include oral administration and parenteral administration such as intravenous administration and the like.

Dosage forms include tablets, powders, granules, syrups, injections, and the like.

For example, liquid preparations that are suitable for oral administration, such as syrups, can be produced using water; saccharides such as sucrose, sorbitol, fructose, and the like; glycols such as polyethylene glycol, propylene glycol, and the like; oils such as sesame oil, olive oil, soybean oil, and the like; antiseptics such as p-hydroxybenzoate esters, and the like; flavors such as strawberry flavor, peppermint, and the like. Tablets, powders, granules, and the like can be produced using excipients such as lactose, glucose, sucrose, mannitol, and the like; disintegrating agents such as starch, sodium alginate, and the like; lubricants such as magnesium stearate, talc, and the like; binders such as polyvinyl alcohol, hydroxypropylcellulose, gelatin, and the like; surfactants such as fatty acid ester, and the like; plasticizers such as glycerol, and the like.

Formulations suitable for parenteral administration preferably contain sterile aqueous agents that are isotonic to the recipient's blood and contain active compounds. For example, in the case of injections, injection solutions are prepared using a carrier containing a salt solution or glucose solution, or a mixture of salt solution and glucose solution, or the like.

For these parenteral agents, one or more of the examples shown for oral agents, such as diluents, antiseptics, flavors, excipients, disintegrators, lubricants, binders, surfactants, plasticizers, and the like, can also be added as supplementary components.

The dosage and the number of doses a peptide of the present invention or a pharmaceutically acceptable salt thereof vary depending on the form of administration, age and body weight of the patient, and characteristics or severity of the symptoms to be treated; in normal oral administration, 0.01 mg to 1 g, or preferably 0.05 to 50 mg is administered once or several times per day for an adult. In parenteral administration, such as intravenous administration or the like, 0.001 mg to 100 mg, or preferably 0.01 mg to 10 mg is administered once or several times per day for an adult. However, the dosage and the number of doses may vary depending on various conditions as mentioned above.

5. Measurement of circulation-modulating activity of C-terminal VGF-derived peptides and pharmaceutically acceptable salts thereof

The circulation-modulating activity of a C-terminal VGF-derived peptide or a pharmaceutically acceptable salt thereof can be confirmed when the following assays show that it has activity to increase intracellular calcium ion concentration or activity to increase blood pressure.

(a) Measurement for the activity of increasing intracellular calcium ion concentration

(i) Use of apoaequorin-expressing transgenic mice
A heart or blood vessel is collected from transgenic mice systemically expressing apoaequorin (WO02/010371 produced by introducing an apoaequorin gene expression vector into fertilized eggs. The obtained heart or blood vessel is cut finely into pieces, suspended in a medium containing coelenterazine, and then incubated to incorporate coelenterazine into the cells to form aequorin (a complex of apoaequorin and coelenterazine). Since aequorin luminesces upon binding to intracellular calcium ions, the relative luminescence level in cells before and after addition of a medium containing the peptide or a pharmaceutically acceptable salt thereof is measured in a luminometer every second over time and is used as an indicator of intracellular calcium ion concentration. Increase in the relative luminescence level due to addition of the peptide or pharmaceutically acceptable salt thereof confirms that the peptide or pharmaceutically acceptable salt thereof has the activity to increase intracellular calcium ion concentration.

(ii) Use of calcium-binding fluorescence reagents
A finely cut heart or blood vessel collected from animal, or a cell line derived from heart or blood vessel is suspended in a buffer containing a calcium ion-binding fluorescence reagent, such as Fura-2, Indo-1, or the like, whose excitation wavelength, fluorescence wavelength, or fluorescence intensity changes depending on the presence or absence of calcium ions, and the suspension is cultured to incorporate the reagent into the cells. The fluorescence excitation wavelength peak shifts from 380 nm to 340 nm as a result of Fura-2 binding to calcium ions. Therefore, the fluorescence intensity ratio between 380 nm excitation and 340 nm excitation is measured with a fluorometer before and after addition of a buffer containing the peptide or a pharmaceutically acceptable salt thereof, and is used as an indicator of intracellular calcium ion concentration. Increase in the fluorescence intensity ratio due to addition of a peptide or a pharmaceutically acceptable salt thereof confirms that this peptide or a pharmaceutically acceptable salt thereof has the activity to increase intracellular calcium ion concentration. The fluorescence wavelength shifts from 480 nm to 400 nm as a result of Indo-1 binding to calcium ions. The fluorescence intensity ratio between 400 nm and 480 nm before and after addition of a buffer containing the peptide or pharmaceutically acceptable salt thereof is measured with a fluorometer, and used as an indicator of intracellular calcium ion concentration. Increase in the fluorescence intensity ratio due to addition of the peptide or a pharmaceutically acceptable salt thereof confirms that this peptide or pharmaceutically acceptable salt thereof has the activity to increase intracellular calcium ion concentration.

(b) Blood pressure increasing activity

Catheters are inserted into the external jugular vein and internal carotid artery of an anesthetized animal such as rat or the like, and arterial pressure is measured continuously by connecting the catheter in the internal carotid artery to a blood pressure monitor. The peptide or pharmaceutically acceptable salt dissolved in physiological saline is administered through the external jugular vein. The peptide or pharmaceutically acceptable salt is confirmed to have the activity to increase blood pressure, when comparison of the arterial pressures before and after administration of the peptide or pharmaceutically acceptable salt shows that the arterial pressure increases due to administration of the peptide or pharmaceutically acceptable salt.

6. Methods of screening for substances that inhibit or promote the C-terminal VGF-derived peptide-induced increase of intracellular calcium ion concentration in cardiac or vascular cells

Screening for substances that inhibit the increase of intracellular calcium ion concentration in cardiac or vascular cells induced by a C-terminal VGF-derived peptide can be carried out by (i)measuring the cellular response elicited when the C-terminal VGF-derived peptide or a pharmaceutically acceptable salt thereof and a test substance are contacted with the cardiac or vascular cells, (ii)comparing this with the cellular response in which the C-terminal VGF-derived peptide or pharmaceutically acceptable salt is contacted with the same cells in the absence of the test substance, and (iii)identifying the test substance as a substance that inhibits the increase of intracellular calcium ion concentration in cardiac or vascular cells induced by the C-terminal VGF-derived peptide, when the cellular response is suppressed in the presence of the test substance.

Similarly, screening for substances that promote the increase of intracellular calcium concentration in cardiac or vascular cells induced by the C-terminal VGF-derived peptide can be carried out by (i)measuring the cellular response elicited when the C-terminal VGF-derived peptide or a pharmaceutically acceptable salt thereof and a test substance are contacted with the cardiac or vascular cells, (ii)comparing this with the cellular response when the C-terminal VGF-derived peptide or pharmaceutically acceptable salt is contacted with the same cells in the absence of the test substance, and (iii)identifying the test substance as a substance that promotes the increase of intracellular calcium ion concentration in cardiac or vascular cells induced by the C-terminal VGF-derived peptide, when the cellular response is promoted in the presence of the test substance.

The cellular response may be any cellular response, for example, an increase in intracellular calcium ion concentration so long as it is a measurable cellular response elicited by the C-terminal VGF-derived peptide when it is contacted with cardiac or vascular cells.

The cardiac or vascular cells used in the above-mentioned screening method may be a cell line derived from a heart or blood vessel, or a finely cut heart or blood vessel collected from an animal, so long as they show cellular responses when contacted with a C-terminal VGF-derived peptide. As intracellular calcium ion concentration can be conveniently measured using a luminometer by measuring the luminescence level in the presence of coelenterazine, it is preferable to use cells obtained by finely cutting a heart or blood vessel collected from a transgenic mouse that is produced by introducing the apoaequorin gene and systemically expresses apoaequorin (WO02/010371)

Substances that promote the increase of intracellular calcium ion concentration in cardiac or vascular cells induced by the C-terminal VGF-derived peptides obtained by the above-mentioned screening method have circulation-modulating activity similar to the C-terminal VGF-derived peptides. Therefore, they can be used as circulation-modulating agents and vasopressors, or therapeutic agents for diseases of the circulatory system including myocardial infarction, ischemic heart disease, cerebral infarction, and the like. Substances that inhibit the increase of intracellular calcium ion concentration in cardiac or vascular cells induced by the C-terminal VGF-derived peptides inhibit activities possessed by C-terminal VGF-derived peptides, such as blood pressure increasing activity, and thus they may be used as antihypertensives.

7. Methods of screening for agonists or antagonists against C-terminal VGF-derived peptide receptors

Screening for agonists or antagonists against C-terminal VGF-derived peptide receptors can be carried out by (i)measuring the level of the C-terminal VGF-derived peptide or a pharmaceutically acceptable salt thereof binding to cardiac or vascular cells, or to a membrane fraction of the cells, when the peptide or pharmaceutically acceptable salt and a test substance are contacted with these cells or their membrane fraction, (ii)comparing this with the level of the C-terminal VGF-derived peptide or pharmaceutically acceptable salt binding to the same cells or membrane fraction of these cells in the absence of the test substance, and (iii)identifying the test substance as an agonist or antagonist against the C-terminal VGF-derived peptide receptor when the binding level of the peptide or pharmaceutically acceptable salt decreases in the presence of the test substance.

The cells described in 5 above can be used as the cardiac or vascular cells. These cells or their cell membrane fraction are suspended in a suitable buffer. The buffer may be any buffer so long as the binding between a C-terminal VGF-derived peptide and the cells or cell membrane fraction is not inhibited, and for example, a phosphate buffer, Tris-HCl buffer, or the like at pH 4 to 10 (or desirably pH 6 to 8) is used. Furthermore, surfactants such as CHAPS, Tween-80, digitonin, deoxycholic acid, or the like, or various proteins such as bovine serum albumin, gelatin, or the like can be added to the buffer to decrease non-specific binding. Furthermore, to suppress degradation of the polypeptides or ligands of the present invention by proteases, a protease inhibitor such as PMSF, leupeptin, E-64, pepstatin, or the like can be added.

Binding experiments are performed by placing a C-terminal VGF-derived peptide labeled with a radioisotope such as $^{125}$I, $^3$H, or the like and having a certain level of radioactivity, together with 10 $\mu$L to 10 mL of a suspension solution of these cells or a cell membrane fraction of these cells. The reaction is carried out at 0 to 50°C, preferably at 4 to 37°C, and for 20 minutes to 24 hours, preferably 30 minutes to 3 hours. The reaction is followed by filtration through a glass fiber filter or the like, and washing with a suitable amount of the same buffer. The radioactivity remaining on the glass fiber filter is measured using a $\gamma$-counter or liquid scintillation counter. This binding level is defined as the total binding level (A). A similar reaction is carried out under conditions in which a large excess of the same but unlabeled compound is added, and this binding level is defined as the non-specific binding level (B). A similar reaction is carried out under conditions in which a test compound is added, and this binding level is defined as C. The rate of binding inhibition of the test substance can be determined by the following equation:

$$\text{Inhibition rate (\%)} = [1 - \{(C - B) / (A - B)\}] \times 100$$

Similarly to C-terminal VGF-derived peptides, receptor agonists of C-terminal VGF-derived peptides that can be obtained by the above-mentioned screening method have circulation-modulating activity. Therefore, they may be used as circulation-modulating agents, vasopressors, and therapeutic agents for diseases of the circulatory system such as myocardial infarction, ischemic heart disease, cerebral infarction, or the like. Furthermore, receptor antagonists of C-terminal VGF-derived peptides inhibit activities possessed by C-terminal VGF-derived peptides such as blood pressure increasing activity, and thus they may be used as antihypertensives.

Hereinafter, Examples of the present invention are described.

[Example 1]

Isolation of VGF-derived peptides and determination of their structures

A human pancreas-derived cell line ($10^8$ cells) was grown until confluent and cultured for six hours in a phenol red-free and serum-free RPMI medium, and the medium was collected. One-fiftieth volume of 1 mol/L hydrochloric acid was added to the supernatant obtained by collecting and centrifuging the medium, and then the sample was extracted using a Sep-Pak C18 cartridge (manufactured by Waters). The cartridge was washed with 0.1 % trifluoroacetic acid (hereinafter abbreviated as TFA), and the sample was eluted with 60% acetonitrile-0.1 % TFA. After freeze-drying the eluate, it was dissolved in 60% acetonitrile-0.1 % TFA, and the peptide fraction was collected by HPLC (HPLC pump L-2100; manufactured by Hitachi). Separation was carried out at a flow rate of 1.5 mL/min using a gel filtration HPLC column (TSKgel G2000SW$_{XL}$, 21.5 mm x 30 cm; manufactured by TOSO) that has been equilibrated with the same solution. This freeze-dried sample was dissolved in 1 mol/L acetic acid, then neutralized with 1 mol/L Tris (pH11), and then warmed at 37°C for one hour in a reduction reaction solution (1mmol/L EDTA, 25 mmol/L dithiothreitol, 0.5 mol/L Tris, pH8.5). Subsequently, iodoacetamide was added at a final concentration of 50 mmol/L, and this was allowed to react in the dark for 15 minutes at room temperature. The reaction was stopped with glacial acetic acid, and desalting was performed using a Sep-Pak C18 cartridge. The desalted and freeze-dried sample was dissolved in 0.1 % TFA, and separation was performed by HPLC (HPLC pump L-6000 (manufactured by Hitachi)) at a flow rate of 50 $\mu$L/min using a reverse phase HPLC column (Vydac Protein & Peptide C18, 1 mm x 15 mm; manufactured by Grace Vydac), and the fractions were collected every 30 seconds. Each fraction was dried under reduced pressure, dissolved in 50% methanol/2% acetic acid, and analyzed using two types of mass spectrometric methods: the matrix assisted laser desorption ionization (MALDI) method and electrospray ionization (ESI) method. In the MALDI method, the above-mentioned sample was applied onto the target plate and then 0.5 μL of a solution of 2.5 mg/mL of α-cyano-4-hydroxycinnamic acid dissolved in 50% ACN-0.1% TFA was added. The plate as subjected to mass spectrometry using a tandem time-of-flight (TOF) mass spectrometer (4700 Proteomics Analyzer; manufactured by Applied Biosystems) and the detected peptides were identified successively. Mass spectrometry using the ESI method was performed using a quadrupole time-of-flight mass spectrometer (Q-Tof2; manufactured by Micromass). The obtained tandem mass spectra were identified using an analysis software (Mascot MS/MS Ion Search; produced by Matrix Science) based on amino-acid sequence databases, NCBI and Swiss-Prot. As a result, six peptides having partial amino acid sequences of human VGF, i.e., Peptide 1 (SEQ ID NO: 27), Peptide 2 (SEQ ID NO: 26), Peptide 3 (SEQ ID NO: 2), Peptide 4 (SEQ ID NO: 5), Peptide 9 (SEQ ID NO: 28), and Peptide 10 (SEQ ID NO: 29) were found. The N termini of Peptide 1 and Peptide 2 were not modified, but their C termini were amidated. Neither of the N and C termini was modified in Peptide 3, Peptide 4, Peptide 9, and Peptide 10. The amino acid sequence (SEQ ID NO: 4) excluding the C-terminal amide of the sequence shown in SEQ ID NO: 27 corresponds to positions 565 to 577 of the amino acid sequence of human VGF (SEQ ID NO: 9); the amino acid sequence (SEQ ID NO: 3) excluding the C-terminal amide of the sequence shown in SEQ ID NO: 26 corresponds to positions 554 to 577 of the amino acid sequence of human VGF (SEQ ID NO: 9); the sequence shown in SEQ ID NO: 2 corresponds to positions 554 to 583 of the amino acid sequence of human VGF (SEQ ID NO: 9); the sequence shown in SEQ ID NO: 5 corresponds to positions 586 to 615 of the amino acid sequence of human VGF (SEQ ID NO: 9); the sequence shown in SEQ ID NO: 28 corresponds to positions 485 to 503 of the amino acid sequence of human VGF (SEQ ID NO: 9); and the sequence shown in SEQ ID NO: 29 corresponds to positions 533 to 552 of the amino acid sequence of human VGF (SEQ ID NO: 9). The amino acid sequence of Peptide 4 (SEQ ID NO: 5) has the same amino acid sequence as peptide V derived from bovine VGF (Endocrinology, 135, 2742-2748 (1994)).

[Example 2]

Preparation of VGF-derived peptides

Peptide 1, Peptide 2, Peptide 3, Peptide 4, Peptide 9 and Peptide 10 discovered in Example 1, and their related peptides: Peptide 5 comprising the amino acid sequence shown in SEQ ID NO: 1 and Peptide 6 comprising the amino acid sequence shown in SEQ ID NO: 6, were chemically synthesized by request (Sigma-Genosys: Peptides 2 to 6; and American Peptide Company Inc.: Peptide 1). The amino acid sequence shown in SEQ ID NO: 1 corresponds to the sequence of positions 554 to 615 in the amino acid sequence of human VGF, and includes all of the amino acid sequences shown in SEQ ID NOS: 2 to 5. The amino acid sequence shown in SEQ ID NO: 6 corresponds to the sequence of positions 556 to 585 in the amino acid sequence of rat VGF, which is a corresponding rat sequence of Peptide 3. Chemical synthesis was performed by a solid phase synthesis method using Nα-Fmoc-protected amino acids on a peptide synthesizer, and the synthesized peptides were purified by reverse phase HPLC.

Peptides 2 to 6 (Sigma-Genosys) were chemically synthesized on an Abacus peptide synthesizer using the TentaGel S RAM resin (manufactured by Rapp Polymere GmBH) for C-terminal amide peptides (Peptide 2), and the TentaGel S PHB resin bound with Nα-Fmoc-protected amino acids for C terminal free peptides (Peptides 3 and 6: TentaGel S PHB-Fmoc-alanine; Peptides 4 and 5: TentaGel S PHB-Fmoc-proline), and Nα-Fmoc-protected amino acids (manufactured by EMD Biosciences). The side chain-protected amino acids for each of the aspartic acid, serine, threonine, glutamic acid, tyrosine, lysine, histidine, arginine and cysteine used are shown below.

Aspartic acid: Fmoc-Asp(OtBu)-OH (Nα-9-fluorenylmethyloxycarbonyl-L-aspartic acid-β-t-butylester)
Serine: Fmoc-Ser(tBu)-OH (Nα-9-fluorenylmethyloxycarbonyl-O-t-butyl-L-serine)
Threonine: Fmoc-Thr(tBu)-OH (Nα-9-fluorenylmethyloxycarbonyl-O-t-butyl-L-threonine)
Glutamic acid: Fmoc-Glu(OtBu)-OH (Nα-9-fluorenylmethyloxycarbonyl-L-glutamic acid-γ-t-butylester)
Tyrosine: Fmoc-Tyr(tBu)-OH (Nα-9-fluorenylmethyloxycarbonyl-O-t-butyl-L-tyrosine)
Lysine: Fmoc-Lys(Boc)-OH (Nα-9-fluorenylmethyloxycarbonyl-ε-t-butyloxycarbonyl-L-lysine)
Histidine: Fmoc-His(Trt)-OH (Nα-9-fluorenylmethyloxycarbonyl-Nim-trityl-L-histidine)
Arginine: Fmoc-Arg(Pbf)-OH
(Nα-9-fluorenylmethyloxycarbonyl-NG-2,2,4,6,7-pentamethyldihydrobenzofuran-5-sulfonyl-L-arginine)
Cysteine: Fmoc-Cys(Trt)-OH: (Nα-9-fluorenylmethyloxycarbonyl-S-trityl-L-cysteine)

A mixture of trifluoroacetic acid (TFA):deionized water:dithiothreitol (powder):triisopropylsilane = 88:5:5:2 was used as the cleavage cocktail for deprotection. Reverse phase HPLC was performed using a Discovery HS C18 column (10 μm, 25 cm x 21.2 cm, manufactured by Sigma-Aldrich) and a solvent system of 0.1% aqueous TFA solution (solution A) and 0.1 % TFA-containing acetonitrile solution (solution B).

Structures of the synthesized Peptides 2 to 6 were confirmed (Sigma-Genosys) by mass spectrometry using the MALDI method on a MALDI-TOF mass spectrometer, Autoflex (manufactured by Brucker). A saturated α-cyano-4-hydroxycinnamic acid (produced by Sigma-Aldrich) solution was prepared with 50% ACN-0.1 % TFA and used as the matrix. The structure of Peptide 1 (American Peptide Company Inc.) was confirmed by mass spectrometry using the MALDI method on a MALDI-TOF mass spectrometer Voyager DE Pro (Applied Biosystems). A two-fold diluted solution of the saturated $\alpha$-cyano-4-hydroxycinnamic acid (produced by Sigma-Aldrich) solution prepared with 50% ACN-0.1 % TFA was used as the matrix. Furthermore, the peptide structures were also confirmed by amino acid analyses (Anal. Biochem., 222, 19 (1994)). Hydrolysis was performed in hydrochloric acid vapor at 110°C for 22 hours using a Pico-Tag Workstation (Waters), and the amino acid composition of the hydrolysis product was determined using an amino acid analyzer L-8500 (manufactured by Hitachi).

[Example 3]

The activity of VGF-derived peptides to increase intracellular calcium ion concentration

Whether Peptides 1 to 5, 9 and 10 synthesized in Example 2 have the activity of increasing intracellular calcium ion concentration was investigated using the organs of transgenic mice introduced with the apoaequorin gene and systemically expressing apoaequorin (hereinafter referred to as apoaequorin-expressing mice). In apoaequorin-expressing mouse cells, light is emitted when apoaequorin binds to a calcium ion in the presence of the luminescent substrate coelenterazine and thus the intracellular calcium ion concentration can be monitored. Patent Document (WO02/010371) discloses the method for producing apoaequorin-expressing mice, method for evaluating biologically active substances that use biological samples derived from the mice, and experimental results of evaluating biologically active peptides using the mouse organs as shown below. More specifically, it is reported that when angiotensin II was added at a final concentration of 1 $\mu$mol/L to each of the organs obtained from the apoaequorin-expressing mice, strong luminescence was observed in the blood vessels, uterus, and adrenal glands; and when bradykinin was added at a final concentration of 10 $\mu$mol/L, strong luminescence was observed in the blood vessels, uterus, and adrenal glands. Therefore, apoaequorin-expressing mice can be used in the evaluation of the physiological activities of novel peptides.
Apoaequorin-expressing mice were produced according to the method disclosed in Reference Example 4 of Patent Document (WO02/010371). The apoaequorin-expressing mice were sacrificed, individual organs including thymus, spleen, bone, blood vessel, heart, kidney, adrenal gland, pancreas, pituitary gland, and uterus, were removed; and each of the organs was cut into small cubes of approximately 1 to 2 mm$^3$. Next, in 5-mL tubes (Rohren-Tubes; manufactured by Sarstedt, No. 55.476), three portions of each of the prepared organs were added to 50$\mu$L of a 10 $\mu$mol/L solution of coelenterazine (manufactured by Molecular Probes) dissolved in RPMI 1640 medium, and these were cultured at 37°C for three hours. After culturing, RPMI 1640 medium was added, and then Peptides 1 to 5 dissolved in RPMI 1640 medium were added 25 seconds after the addition of RPMI 1640 until each peptide had final concentrations of 1 $\mu$mol/L and of 5 $\mu$mol/L, and the relative luminescence levels were measured every second immediately after the addition of RPMI 1640 medium by using a luminometer (AutoLumat LB953, manufactured by Berthold).
As a result, luminescence was observed in the thymus, heart (Fig. 1), pituitary gland and uterus for Peptide 1; in the thymus, spleen, bone, blood vessel (Fig. 2), heart (Fig. 3) and uterus for Peptide 2; in the thymus, spleen, bone, blood vessel, heart (Fig. 4), kidney, adrenal gland, pituitary gland and uterus for Peptide 3; in the thymus, spleen, blood vessel (Fig. 5) and pituitary gland for Peptide 4; in the heart (Fig. 6) for Peptide 5; in the blood vessel (Fig. 7) for Peptide 9; and in the heart (Fig. 8) and blood vessel (Fig. 9) for peptide 10. From the above, it was found that Peptides 1 to 5 and Peptides 9 and 10 have an activity of increasing intracellular calcium ion concentration in cells of the heart or blood vessels which are organs of the circulatory system.

[Example 4]

Effects of Peptide 2 on rat blood pressure

Wistar rats (male, 10-weeks old, 250 to 300 g) were anesthetized by intraperitoneally administering Pentobarbital at 50 mg/kg body weight. Catheters were inserted into the external jugular vein and internal carotid artery of the anesthetized rats; the catheter in the internal carotid artery was connected to a blood pressure monitor; 300 nmol of Peptide 2 dissolved in saline (100 $\mu$L) was administered from the external jugular vein by bolus injection; and blood pressure was measured continuously. The change in average arterial blood pressure after peptide administration is shown in Fig. 10. As shown in Fig. 10, administration of Peptide 2 showed an obvious increase in blood pressure.

[Example 5]

Antibody production with Peptide 1 as antigen

(1) Animal immunization and antiserum preparation

To conjugate Peptide 1 to a carrier protein, Peptide 7 comprising the amino acid sequence shown in SEQ ID NO: 7, which is a sequence in which a cysteine residue is added to the N terminus of Peptide 1, was chemically synthesized by request as in Example 1 (American Peptide Company Inc.). 4.9 mg of Peptide 7 was covalently bonded with 10 mg of maleimide-activated keyhole limpet hemocyanin (Inject Activated mcKLH; manufactured by Pierce) via the cysteine residue. The covalent bonding reaction was performed according to the manual provided by Pierce. The obtained conjugate between Peptide 7 and KLH was dialyzed against physiological saline, and this was used as an antigen. The antigen was dispensed and stored at -35°C until use. One milliliter of the obtained antigen solution in physiological saline (equivalent to approximately 200 $\mu$g of Peptide 7) was mixed with an equivalent amount of the Freund's complete adjuvant to prepare a stable emulsion, and intradermally administered nine times to a male rabbit (New Zealand white rabbit) for immunization in three-week intervals. After repeated administration, antibody titer was measured, serum was prepared from rabbits showing an increase in antibody titer, and this was used as the antiserum.

(2) Antibody titer measurement

Antibody titer was measured by radioimmunoassay (RIA) as indicated below. Namely, 100 $\mu$L of RIA buffer containing Peptide 1 labeled with a specified amount of [125I] (approximately 20000 cpm, 500 to 550 Bq, approximately 10 fmol of peptide) was added to 100 $\mu$L of antiserum sequentially diluted with RIA buffer (25 mmol/L EDTA, 80 mmol/L sodium chloride, 0.05% sodium azide, 0.5% N-ethylmaleimide-treated BSA, 50 mmol/L sodium phosphate buffer containing 0.5% TritonX-100 (pH7.4)) in a polystyrene tube, and this was incubated at 4°C for 40 hours to link the antibody in the antiserum to the labeled Peptide 1. To measure non-specific binding, reactions using an antiserum-free RIA buffer solution instead of the antiserum were carried out as control. After incubation, 100 $\mu$L of a 1 % bovine $\gamma$-globulin (manufactured by Sigma-Aldrich) solution (50 mmol/L sodium phosphate buffer containing 80 mmol/L sodium chloride and 0.05% sodium azide (pH7.4)) was added and mixed; then 500 $\mu$L of a 23% polyethylene glycol #6000 (manufactured by Nakalai Tesque) solution (50 mmol/L sodium phosphate buffer containing 80 mmol/L sodium chloride and 0.05% sodium azide (pH7.4)) was further added and mixed. This was left on ice for ten minutes or more, and then centrifuged for 15 minutes at 3000 rpm to precipitate the immune complex. Supernatant containing the unbound [125I]-labeled Peptide 1 was removed by aspiration, and radioactivity A (cpm) of the precipitate was measured using a $\gamma$-counter. Radioactivity N was similarly measured in the tubes for non-specific binding reaction (non-specific binding level; cpm), and value N for non-specific binding was subtracted from the precipitate's radioactivity value A, and the value obtained was defined as the specific binding level of the antiserum. Radioactivity T (cpm) of the 100 $\mu$L RIA buffer containing the [125I]-labeled Peptide 1 used in the reaction was measured using a $\gamma$-counter, and the percentage ratio (X) of specific binding level to radioactivity of added antigenic peptide was determined by the following equation. Dilution ratio of antiserum was plotted against X, and the inverse of the dilution ratio at which X becomes 30% was used as the indictor for antibody titer.

$$X \, (\%) = \{(A - N)/T\} \times 100$$

[125I] labeling of the antigenic peptide was carried out using the lactoperoxidase method by labeling the tyrosine residue introduced to the N-terminus of the antigenic peptide. Specifically, 10 $\mu$g of Peptide 1 was dissolved in 25 $\mu$L of 0.4 mol/L sodium acetate (pH5.6), then 10 $\mu$L of 0.1mol/L sodium acetate containing 200 ng of lactoperoxidase (pH5.6), 5 $\mu$L of 3.7 MBq/$\mu$L Na 125I (18.5 MBq), and 5 $\mu$L of 0.002% hydrogen peroxide were added, and this was reacted with stirring at 30°C for ten minutes. In addition, 5 $\mu$L of 0.002% hydrogen peroxide was added, and reacted with stirring at 30°C for ten minutes. Five hundred microliter of water was added and fractions of the labeled peptides were collected by C18 reverse phase HPLC using a solvent system of 10% to 60% ACN gradient/0.1 % TFA. The peptide solutions were diluted with 60% ACN-0.1% TFA, dispensed into aliquots, and then stored at -85°C.

(3) Binding specificity of antisera

Of the obtained antisera, the above-mentioned antiserum showing an antibody titer of 9 x 10^5 (specifically, an antiserum in which 30% of the antigenic peptide added in the above-mentioned RIA showed binding activity even at 9 x 10^5-fold dilution) was used to examine the binding specificity against the six types of VGF-derived peptides using RIA.

Namely, to 100 μL of antiserum diluted $9\times10^5$ folds with RIA buffer, 100 μL of RIA buffer containing a fixed amount of [$^{125}$I]-labeled Peptide 1 (approximately 20000 cpm, 500-550 Bq, approximately 10 fmol in terms of peptide amount) and 100 μL of RIA buffer solutions each containing a sequentially diluted VGF-derived peptide were added, this was incubated at 4°C for 40 hours, and the labeled Peptide 1 and VGF-derived peptide were competitively bound to the antibody in the antiserum. As controls, reactions that use RIA buffer instead of the antiserum to measure non-specific binding, and reactions without that use RIA buffer instead of VGF-derived peptide solutions to measure the maximum binding level were carried out. Reactions for non-specific binding were performed in quadruplicates, and the other reactions were performed in duplicates, and for each of the reactants, the immune complex was precipitated as in the above-mentioned antibody titer measurements, and its radioactivity (cpm) was measured. The average radioactivity of non-specific binding reaction is defined as non-specific binding level N, the radioactivity value for each VGF-derived peptide addition reaction is defined as Y, and the radioactivity value of maximum binding reaction is defined as Z. The percentage ratio (B/B$_0$) of antiserum specific binding level (B) with peptide addition to the maximum binding level (B$_0$), was determined using the following formula.

$$B/B_0\ (\%) = \{(Y - N)/(Z - N)\} \times 100$$

When the antiserum also binds to the added peptide, the added peptide competitively inhibits the binding of antiserum to $^{125}$I-labeled antigenic peptide in an amount-dependent manner; therefore, the specific binding level of antiserum to $^{125}$I-labeled antigenic peptide is decreased when the peptide is added. Accordingly, the amount of peptide that achieves 50% BB$_0$, more specifically, the amount of peptide that yields 50% inhibition of the maximum binding level, was used as an indicator of the peptide binding activity to the antiserum. A smaller amount of peptide required to yield 50% inhibition indicates a greater binding activity.

Peptides 1 to 3 prepared in Example 2 and Peptide 8 comprising the amino acid sequence shown in SEQ ID NO: 8 were used as VGF-derived peptides. The sequence shown in SEQ ID NO: 8 is the sequence of positions 606 to 615 of the human VGF amino acid sequence, and does not include the amino acid sequence of Peptide 1. The amounts of peptide/tube used for performing the measurements were 0.5, 1, 2, 4, 8, 16, 32, 64, 128, 256, 512 and 1024 fmol for Peptide 1; 1, 2, 4, 8, 16, 32, 64, 128, 256, 512 and 1024 fmol for Peptide 2; 1, 3.3, 10, 33, 100, 333, 1000, 3300, 10,000, 33,000, 100,000, 333,000 and 1,000,000 fmol for Peptide 3; and 1,000, 10,000, 100,000 and 1,000,000 fmol for Peptide 8.

As shown in Fig. 11, Peptides 1 and 2 showed the same degree of quantity-dependent inhibition of antiserum binding, and the amount of Peptide 1 for 50% inhibition was 7.5 fmol. Peptide 3 also showed dose-dependent inhibition of antiserum binding but the inhibition was weak, and to show the same degree of inhibition as Peptides 1 and 2, an amount of approximately 10,000 folds was required. Peptide 8 did not show any inhibition. This antiserum binds strongly to the Peptide 1 antigen and shows a same degree of strong binding towards Peptide 2, which has a structure that includes Peptide 1. It binds weakly to Peptide 3 (1/10000 of the Peptide 1 binding), and does not bind to Peptide 8 at all. Therefore, this antiserum is an antibody that specifically binds to Peptides 1 and 2. Binding was examined for non-VGF peptides, such as angiotensin II, calcitonin gene-related peptide, Leu-enkephalin, neuromedin U-8, substance K, vasopressin, Met-enkephalin-Arg-Gly-Leu, adrenomedulin, calcitonin, PHI-27, corticotropin-releasing factor, PAMP-20, and calcitonin receptor-stimulating peptide, in the same way as described above using 1, 10, and 100 pmols of the peptides, but none of the peptides showed inhibition at concentrations of 1, 10, and 100 pmol, and thereby confirmed that the antibody binds specifically to Peptides 1 and 2.

Peptide 3 has a structure that includes the amino acid sequence of Peptide 1 but does not have the C terminal amide of Peptide 1, and Peptide 8 has a structure that does not include the amino acid sequence of Peptide 1. Therefore, this antiserum was considered to be an antibody that binds to an epitope present in the sequence of Peptide 1, in which the C terminus of the amino acid sequence shown in SEQ ID NO: 4 has been amidated (SEQ ID NO: 27), and in particular, an antibody that binds to an epitope present in the region comprising the C terminal amide of this sequence.

Industrial Applicability

The present invention provides novel peptides having circulation-modulating activity. Since these peptides have circulation-modulating activity, they are useful as circulation-modulating agents and vasopressors, and can be used for treating diseases of the circulatory system such as myocardial infarction, ischemic heart disease, cerebral infarction, or the like.

[Sequence Listing Free Text]

SEQ ID NO: 1 - Inventors: Yamasaki, Motoo; Takahashi, Noriyuki; Minamino, Naoto; Inventors: Sasaki, Kazuki; Takao, Toshifumi; Satomi, Yoshinori
SEQ ID NO: 7 - Synthetic Peptide 7
SEQ ID NO: 10 - DNA encoding the amino acid sequence shown in SEQ ID NO: 1
SEQ ID NO: 11 - DNA encoding the amino acid sequence shown in SEQ ID NO: 2
SEQ ID NO: 12 - DNA encoding the amino acid sequence shown in SEQ ID NO: 3
SEQ ID NO: 13 - DNA encoding the amino acid sequence shown in SEQ ID NO: 4
SEQ ID NO: 17 - VGF-derived peptide consensus sequence
SEQ ID NO: 18 - VGF-derived peptide consensus sequence
SEQ ID NO: 19 - VGF-derived peptide consensus sequence
SEQ ID NO: 20 - VGF-derived peptide consensus sequence
SEQ ID NO: 21 - peptide based on a corresponding rat VGF sequence of Peptide 2
SEQ ID NO: 22 - peptide based on a corresponding rat VGF sequence of Peptide 1
SEQ ID NO: 23 - VGF-derived peptide consensus sequence
SEQ ID NO: 24 - VGF-derived peptide consensus sequence
SEQ ID NO: 34 - DNA encoding the amino acid sequence shown in SEQ ID NO: 28
SEQ ID NO: 35 - DNA encoding the amino acid sequence shown in SEQ ID NO: 29

## Claims

1. A peptide of any one of (a) to (e) below or a pharmaceutically acceptable salt thereof:

   (a) a peptide comprising the amino acid sequence of any one of SEQ ID NOS: 1 to 4, 28 and 29 (but excluding peptides comprising the amino acid sequence of SEQ ID NOS: 9, 30, 31, 32 or 33);
   (b) a peptide comprising an amino acid sequence with substitution, deletion, or addition of one to five amino acids in the amino acid sequence of any one of SEQ ID NOS: 1 to 4, 28 and 29, wherein the peptide has an activity of increasing the intracellular calcium ion concentration of cardiac or vascular cells;
   (c) a peptide comprising an amino acid sequence having 90% or higher homology to the amino acid sequence of any one of SEQ ID NOS: 1 to 4, 28 and 29, wherein the peptide has an activity of increasing the intracellular calcium ion concentration of cardiac or vascular cells;
   (d) a peptide represented by the following formula (I) (but excluding peptides comprising the sequences of SEQ ID NOS: 14 to 16),

   $$Z^1\text{-A-}Z^2 \qquad (I)$$

   (wherein, $Z^1$ represents a hydrogen atom or a peptide residue of any sequence comprising one to eleven amino acids, A represents a peptide residue comprising the amino acid sequence of SEQ ID NO: 17, and $Z^2$ represents an amino group or a peptide residue of any sequence comprising one to 38 amino acids); and
   (e) a peptide represented by the following formula (II)

   $$R^1\text{-B-}R^2 \qquad (II)$$

   (wherein, $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and B represents a peptide residue of the peptide of any one of the above-mentioned (a) to (d)).

2. The peptide of claim 1 or a pharmaceutically acceptable salt thereof, wherein the peptide is a peptide of any one of:

   (a) a peptide comprising the amino acid sequence of SEQ ID NO: 1;
   (b) a peptide comprising the amino acid sequence of SEQ ID NO: 2;
   (c) a peptide comprising the amino acid sequence of SEQ ID NO: 28;
   (d) a peptide comprising the amino acid sequence of SEQ ID NO: 29;
   (e) a peptide wherein in formula (II) of claim 1, $R^1$ is a hydrogen atom, B is a peptide residue comprising the amino acid sequence of SEQ ID NO: 3, and $R^2$ is unsubstituted amino; and
   (f) a peptide wherein in formula (II) of claim 1, $R^1$ is a hydrogen atom, B is a peptide residue comprising the amino acid sequence of SEQ ID NO: 4, and $R^2$ is unsubstituted amino.

3. A DNA encoding any one of the peptides of (a) to (c) of claim 1.

4. A recombinant vector obtainable by incorporating the DNA of claim 3 into a vector.

5. A transformant obtainable by introducing the recombinant vector of claim 4 into a host cell.

6. A method for producing a peptide, which comprises culturing the transformant of claim 5 in a medium so as to produce and accumulate said peptide in the culture, and recovering said peptide from the culture.

7. An antibody that binds to an epitope present in the amino acid sequence of SEQ ID NO: 4 or a sequence wherein the C terminus of the amino acid sequence of SEQ ID NO: 4 is amidated.

8. A method of detecting or quantifying the peptide of claim 1 or 2, which comprises using the antibody of claim 7.

9. A circulation-modulating agent comprising as an active ingredient at least one peptide selected from (a) to (f) below or a pharmaceutically acceptable salt thereof:

> (a) a peptide comprising the amino acid sequence of any one of SEQ ID NOS: 1 to 5, 28 and 29;
> (b) a peptide comprising an amino acid sequence with substitution, deletion, or addition of one to five amino acids in the amino acid sequence of any one of SEQ ID NOS: 1 to 5, 28 and 29, wherein the peptide has an activity of increasing the intracellular calcium ion concentration of cardiac or vascular cells;
> (c) a peptide comprising an amino acid sequence having 90% or higher homology to the amino acid sequence of any one of SEQ ID NOS: 1 to 5, 28 and 29, wherein the peptide has an activity of increasing the intracellular calcium ion concentration of cardiac or vascular cells; and
> (d) a peptide represented by formula (I) of claim 1;
> (e) a peptide comprising the amino acid sequence of SEQ ID NO: 23, or a peptide comprising an amino acid sequence wherein any amino acid sequence comprising one to 32 amino acids has been added to the N terminus of the amino acid sequence of SEQ ID NO: 23;
> (f) a peptide represented by the following formula (III)

$$R^3\text{-}C\text{-}R^4 \qquad\qquad (III)$$

> (wherein, $R^3$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^4$ represents hydroxy, substituted or unsubstituted alkoxy, or substituted or unsubstituted amino; and C represents a peptide residue of the peptide of any one of the above-mentioned (a) to (e)).

10. A method of screening for a substance that inhibits peptide-induced increase of intracellular calcium ion concentration in cardiac or vascular cells, which comprises:

> measuring the cellular response elicited when a test substance and the peptide of any one of (a) to (f) of claim 9 or a pharmaceutically acceptable salt thereof are contacted with cardiac or vascular cells; and
> identifying the test substance as a substance that inhibits the peptide-induced increase of intracellular calcium ion concentration in cardiac or vascular cells, if the test substance suppresses the cellular response compared to the cellular response when said peptide or a pharmaceutically acceptable salt thereof is contacted with said cells in the absence of the test substance.

11. A method of screening for a substance that promotes peptide-induced increase of intracellular calcium ion concentration in cardiac or vascular cells, which comprises:

> measuring the cellular response elicited when a test substance and the peptide of any one of (a) to (f) of claim 9 or a pharmaceutically acceptable salt thereof are contacted with cardiac or vascular cells; and
> identifying the test substance as a substance that promotes the peptide-induced increase of intracellular calcium ion concentration in cardiac or vascular cells, if the test substance promotes the cellular response as compared to the cellular response when said peptide or a pharmaceutically acceptable salt thereof is contacted with said cells in the absence of the test substance.

12. A method of screening for a peptide receptor agonist or antagonist, the method comprising:

measuring the binding level of said peptide or a pharmaceutically acceptable salt thereof to cardiac or vascular cells, or a membrane fraction of said cells, when a test substance and the peptide of any one of (a) to (f) of claim 9 or a pharmaceutically acceptable salt thereof are contacted with said cells or cell membrane fraction; and identifying the test substance as an agonist or antagonist for the receptor of said peptide if the test substance causes a decrease in the binding level of said peptide or a pharmaceutically acceptable salt thereof as compared to when said peptide or a pharmaceutically acceptable salt thereof is contacted with said cells or cell membrane fraction in the absence of the test substance.

# FIG. 1

# FIG. 2

# FIG. 3

# FIG. 4

# FIG. 5

# FIG. 6

# FIG. 7

# FIG. 8

# FIG. 9

# FIG. 10

# FIG. 11

**INTERNATIONAL SEARCH REPORT**

| | International application No. |
|---|---|
| | PCT/JP2006/314969 |

**A. CLASSIFICATION OF SUBJECT MATTER**
*C12N15/09*(2006.01)i, *A61K38/00*(2006.01)i, *A61K38/22*(2006.01)i,
*A61P9/10*(2006.01)i, *A61P43/00*(2006.01)i, *C07K7/08*(2006.01)i,
*C07K14/475*(2006.01)i, *C07K16/24*(2006.01)i, *C12N1/15*(2006.01)i,
According to International Patent Classification (IPC) or to both national classification and IPC

**B. FIELDS SEARCHED**

Minimum documentation searched (classification system followed by classification symbols)
C12N15/09, A61K38/00, A61K38/22, A61P9/10, A61P43/00, C07K7/08,
C07K14/475, C07K16/24, C12N1/15, C12N1/19, C12N1/21, C12N5/10, C12P21/02,
C12Q1/02, G01N33/15, G01N33/50

Documentation searched other than minimum documentation to the extent that such documents are included in the fields searched

Electronic data base consulted during the international search (name of data base and, where practicable, search terms used)
CA/MEDLINE/BIOSIS/REGISTRY/WPIDS(STN), SwissProt/PIR/GeneSeq

**C. DOCUMENTS CONSIDERED TO BE RELEVANT**

| Category* | Citation of document, with indication, where appropriate, of the relevant passages | Relevant to claim No. |
|---|---|---|
| X | WO 01/64835 A1 (HYSEQ, INC.), 07 June, 2001 (07.06.01), SEQ ID NO.26831 & EP 1683809 A | 1,3-6 |
| X | Canu, Nadia; Possenti, Roberta; Ricco, Angela Serena; Rocchi, Mariano; Levi, Andrea, Cloning, structural organization analysis, and chromosomal assignment of the human gene for the neurosecretory protein VGF, Genomics (1997), 45(2), 443-446, Fig. 1 | 1,3-6 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 August, 2006 (11.08.06) | 29 August, 2006 (29.08.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

| | INTERNATIONAL SEARCH REPORT | International application No. |
|---|---|---|
| | | PCT/JP2006/314969 |

C (Continuation).   DOCUMENTS CONSIDERED TO BE RELEVANT

| Category* | Citation of document, with indication, where appropriate, of the relevant passages | Relevant to claim No. |
|---|---|---|
| X | Salton, Stephen R.J.; Fischberg, Daniel J.; Dong, Ke Wen, Structure of the gene encoding VGF, a nervous system-specific mRNA that is rapidly and selectively induced by nerve growth factor in PC12 cells, Molecular and Cellular Biology (1991), 11(5), 2335-49, Fig. 9 | 1,3-6 |
| X | Trani, E.; Giorgi, A.; Canu, N.; Amadoro, G.; Rinaldi, A.M.; Halban, P.A.; Ferri, G.L.; Possenti, R.; Schinina, M.E.; Levi, A., Isolation and characterization of VGF peptides in rat brain. Role of PC1/3 and PC2 in the maturation of VGF precursor, Journal of Neurochemistry (2002), 81(3), 565-574, VGF-6, VGF-10 | 1,3-6 |
| X | JP 2003-505428 A  (Amgen, Inc.), 12 February, 2003 (12.02.03), SEQ ID NO.6 & WO 01/007074 A1 | 1,3-6 |
| A | JP 2004-531250 A  (Biovision AG.), 14 October, 2004 (14.10.04), Full text & WO 2002/082075 A1 | 1-12 |

Form PCT/ISA/210 (continuation of second sheet) (April 2005)

**INTERNATIONAL SEARCH REPORT**

International application No.

PCT/JP2006/314969

Continuation of A. CLASSIFICATION OF SUBJECT MATTER
 (International Patent Classification (IPC))

$C12N1/19$(2006.01)i, $C12N1/21$(2006.01)i, $C12N5/10$(2006.01)i,
$C12P21/02$(2006.01)i, $C12Q1/02$(2006.01)i, $G01N33/15$(2006.01)i,
$G01N33/50$(2006.01)i

(According to International Patent Classification (IPC) or to both national
classification and IPC)

Form PCT/ISA/210 (extra sheet) (April 2005)

**INTERNATIONAL SEARCH REPORT**

| International application No. |
| --- |
| PCT/JP2006/314969 |

**Box No. II**      **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**      **Observations where unity of invention is lacking (Continuation of item 3 of first sheet)**

This International Searching Authority found multiple inventions in this international application, as follows:
    It appears that not all the peptides of the invention of this application have a novel amino acid sequence in common.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    the    ☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0107477 A
- WO 0282075 A
- JP 58110600 A
- JP 3022979 A
- JP 248394288 B
- JP 2227075 A
- JP 63000299 A
- JP 59140885 A
- JP 60070080 A

- WO 9400977 A
- JP 60251887 A
- JP 2606856 B
- JP 2517813 B
- JP 1102096 A
- JP 5336963 A
- WO 9423021 A
- WO 02010371 A

**Non-patent literature cited in the description**

- *Science, (USA,* 1985, vol. 229 (4711), 393-395
- *Genomics, (USA,* 1997, vol. 45 (2), 443-446
- *Neuron, (USA,* 1999, vol. 23 (3), 537-548
- *Cellular and Molecular Neurobiology, (USA,* 2004, vol. 24 (4), 517-533
- *Journal ofNeurochemistry, (UK,* 2002, vol. 81 (3), 565-574
- *European Journal of Neuroscience, (France,* 2004, vol. 20 (11), 3035-3040
- *Journal of Chromatography B: Biomedical Sciences and Applications, (Holland,* 2001, vol. 754 (2), 357-367
- *Endocrinology, (USA,* 1994, vol. 135 (6), 2742-2748
- *Endocrinology, (USA,* 1999, vol. 140 (8), 3727-3735
- *The EMBO Journal, (UK,* 1989, vol. 8 (8), 2217-2223
- **IZUMIYA, N. ; KATO, T. et al.** *Fundamentals and Experiments of Peptide Synthesis,* 1985
- Organic Synthesis (Yuki Gosei) IV, Acid, Amino acid and Peptide. **AIMOTO, S. et al.** Experimental Chemical Course. 1999, vol. 22
- *Int. J. Pept. Protein Res.,* 1990, vol. 35, 161-214
- **FIELDS, G. B.** Solid-Phase Peptide Synthesis, Methods in Enzymology. Academic Press, 1997, vol. 289
- **PENNINGTON, M. W. ; DUNN, B. M.** Peptide Synthesis Protocols, Methods in Molecular Biology. Humana Press, 1994, vol. 35
- **IZUMIYA, N. et al.** Peptide Gosei no Kiso to Jikken. Maruzen, 1985
- Zoku Iyakuhin no Kaihatsu. Peptide Synthesis. Hirokawa Shoten, 1991, vol. 14

- Chemistry of Protein IV-Chemical Modification and Peptide Synthesis. Seikagaku Jikken Koza. Tokyo Kagaku Dojin, vol. 1
- **OHNO, M. et al.** *Seibutsukagaku Jikkenho,* vol. 12 and 1
- Tanpakushitsu no Kagaku Shushoku. Japan Scientific Societies Press, 1981, vol. I and II
- Molecular Cloning: A Laboratory Manual. Cold Spring Harbor Laboratory Press, 2001
- *Agric. Biol. Chem.,* 1984, vol. 48, 669
- *Agric. Biol. Chem.,* 1989, vol. 53, 277
- *Proc. Natl. Acad. Sci., USA,* 1985, vol. 82, 4306
- *Proc. Natl. Acad. Sci. USA,* 1972, vol. 69, 2110
- *Gene,* 1982, vol. 17, 107
- *Molecular & General Genetics,* 1979, vol. 168, 111
- *Methods. in Enzymol.,* 1990, vol. 194, 182
- *Proc. Natl. Acad. Sci. USA,* 1978, vol. 75, 1929
- *J. Bacteriology.,* 1983, vol. 153, 163
- *Cytotechnology,* 1990, vol. 3, 133
- *Nature,* 1987, vol. 329, 840
- *J. Biochem.,* 1987, vol. 101, 1307
- *Proc. Natl. Acad. Sci. USA,* 1987, vol. 84, 7413
- *Virology,* 1973, vol. 52, 456
- Current Protocols in Molecular Biology; Baculovirus Expression Vectors, A Laboratory Manual. W.H. Freeman and Company, 1992
- *Bio/Technology,* 1988, vol. 6, 47
- Baculovirus Expression Vectors, A Laboratory Manual. W. H. Freeman and Company, 1992
- *The Journal of the American Medical Association,* 1967, vol. 199, 519

- *Science,* 1952, vol. 122, 501
- *Virology,* 1959, vol. 8, 396
- *Proceeding of the Society for the Biological Medicine,* 1950, vol. 73, 1
- *Nature,* 1962, vol. 195, 788
- *J. Biol. Chem.,* 1989, vol. 264, 17619
- *Proc. Natl. Acad. Sci., USA,* 1989, vol. 86, 8227
- *Genes Develop.,* 1990, vol. 4, 1288
- **IGAKU SHOIN.** *Enzyme-linked Immunosorbent Assay,* 1976
- Antibodies - A Laboratory Manual. Cold Spring Harbor Laboratory, 1988
- Antibodies, A Laboratory manual. Cold Spring Harbor Laboratory, 1988
- *Curr. Topics. Microbiol. Immunol.,* 1978, vol. 81, 1
- *Europ. J. Immunol.,* 1976, vol. 6, 511
- *Nature,* 1978, vol. 276, 269
- *J. Immunol.,* 1979, vol. 123, 1548
- *Nature,* 1975, vol. 256, 495
- Tan-Clone-Kotai-Manual. Kodansha-Scientific, 1987
- Zoku-Seikagaku Jikken Kouza 5, Meneki-seikagaku Kenkyuho. Tokyo Kagaku Dojin, 1986
- *Endocrinology,* 1994, vol. 135, 2742-2748
- *Anal. Biochem.,* 1994, vol. 222, 19

SEQUENCE LISTING

<110>  Kyowa Hakko Kogyo Co., Ltd.
       Japan as Represented by the President of National Cardiovascular Center
       Osaka University

<120>  Novel peptide

<130>  1799

<150>  JP2005-221635
<151>  2005-07-29

<160>  35

<170>  PatentIn version 3.2

<210>  1
<211>  62
<212>  PRT
<213>  Homo sapiens

<220>
<223>  Inventor: Yamasaki, Motoo; Takahashi, Noriyuki; Minamino, Naoto;
       Inventor: Sasaki, Kazuki; Takao, Toshifumi; Satomi, Yoshinori

<400>  1

Thr Leu Gln Pro Pro Ser Ala Leu Arg Arg Arg His Tyr His His Ala
1               5                   10                  15

Leu Pro Pro Ser Arg His Tyr Pro Gly Arg Glu Ala Gln Ala Arg Arg
                20                  25                  30

Ala Gln Glu Glu Ala Glu Ala Glu Glu Arg Arg Leu Gln Glu Gln Glu
35            40            45

Glu Leu Glu Asn Tyr Ile Glu His Val Leu Leu Arg Arg Pro
50            55            60

<210> 2
<211> 30
<212> PRT
<213> Homo sapiens

<400> 2

Thr Leu Gln Pro Pro Ser Ala Leu Arg Arg Arg His Tyr His His Ala
1            5            10            15

Leu Pro Pro Ser Arg His Tyr Pro Gly Arg Glu Ala Gln Ala
20            25            30

<210> 3
<211> 24
<212> PRT
<213> Homo sapiens

<400> 3

Thr Leu Gln Pro Pro Ser Ala Leu Arg Arg Arg His Tyr His His Ala
1            5            10            15

Leu Pro Pro Ser Arg His Tyr Pro
20

<210> 4
<211> 13
<212> PRT
<213> Homo sapiens

<400> 4

His Tyr His His Ala Leu Pro Pro Ser Arg His Tyr Pro
1               5                   10


<210> 5
<211> 30
<212> PRT
<213> Homo sapiens

<400> 5

Ala Gln Glu Glu Ala Glu Ala Glu Glu Arg Arg Leu Gln Glu Gln Glu
1               5                   10                  15


Glu Leu Glu Asn Tyr Ile Glu His Val Leu Leu Arg Arg Pro
            20              25                  30


<210> 6
<211> 30
<212> PRT
<213> Rattus norvegicus

<400> 6

Thr Leu Gln Pro Pro Ala Ser Ser Arg Arg Arg His Phe His His Ala

Leu Pro Pro Ala Arg His His Pro Asp Leu Glu Ala Gln Ala

<210> 7
<211> 14
<212> PRT
<213> Artificial

<220>
<223> synthetic peptide 7

<220>
<221> MOD_RES
<222> (14)..(14)
<223> AMIDATION

<400> 7

Cys His Tyr His His Ala Leu Pro Pro Ser Arg His Tyr Pro

<210> 8
<211> 10
<212> PRT
<213> Homo sapiens

<400> 8

Tyr Ile Glu His Val Leu Leu Arg Arg Pro

<210> 9
<211> 615
<212> PRT
<213> Homo sapiens

<400> 9

Met Lys Ala Leu Arg Leu Ser Ala Ser Ala Leu Phe Cys Leu Leu Leu
1               5                   10                  15

Ile Asn Gly Leu Gly Ala Ala Pro Pro Gly Arg Pro Glu Ala Gln Pro
            20                  25                  30

Pro Pro Leu Ser Ser Glu His Lys Glu Pro Val Ala Gly Asp Ala Val
            35                  40                  45

Pro Gly Pro Lys Asp Gly Ser Ala Pro Glu Val Arg Gly Ala Arg Asn
            50                  55                  60

Ser Glu Pro Gln Asp Glu Gly Glu Leu Phe Gln Gly Val Asp Pro Arg
65                  70                  75                  80

Ala Leu Ala Ala Val Leu Leu Gln Ala Leu Asp Arg Pro Ala Ser Pro
                85                  90                  95

Pro Ala Pro Ser Gly Ser Gln Gln Gly Pro Glu Glu Glu Ala Ala Glu
            100                 105                 110

Ala Leu Leu Thr Glu Thr Val Arg Ser Gln Thr His Ser Leu Pro Ala
        115                 120                 125

Pro Glu Ser Pro Glu Pro Ala Ala Pro Pro Arg Pro Gln Thr Pro Glu
        130                 135                 140

Asn Gly Pro Glu Ala Ser Asp Pro Ser Glu Glu Leu Glu Ala Leu Ala
145                 150                 155                 160

Ser Leu Leu Gln Glu Leu Arg Asp Phe Ser Pro Ser Ser Ala Lys Arg
                165                 170                 175

Gln Gln Glu Thr Ala Ala Ala Glu Thr Glu Thr Arg Thr His Thr Leu
                180                 185                 190

Thr Arg Val Asn Leu Glu Ser Pro Gly Pro Glu Arg Val Trp Arg Ala
        195                 200                 205

Ser Trp Gly Glu Phe Gln Ala Arg Val Pro Glu Arg Ala Pro Leu Pro
        210                 215                 220

Pro Pro Ala Pro Ser Gln Phe Gln Ala Arg Met Pro Asp Ser Gly Pro
225                 230                 235                 240

Leu Pro Glu Thr His Lys Phe Gly Glu Gly Val Ser Ser Pro Lys Thr
                245                 250                 255

His Leu Gly Glu Ala Leu Ala Pro Leu Ser Lys Ala Tyr Gln Gly Val
     260          265         270

Ala Ala Pro Phe Pro Lys Ala Arg Arg Pro Glu Ser Ala Leu Leu Gly
     275          280         285

Gly Ser Glu Ala Gly Glu Arg Leu Leu Gln Gln Gly Leu Ala Gln Val
     290          295         300

Glu Ala Gly Arg Arg Gln Ala Glu Ala Thr Arg Gln Ala Ala Ala Gln
305          310         315         320

Glu Glu Arg Leu Ala Asp Leu Ala Ser Asp Leu Leu Leu Gln Tyr Leu
     325          330         335

Leu Gln Gly Gly Ala Arg Gln Arg Gly Leu Gly Gly Arg Gly Leu Gln
     340          345         350

Glu Ala Ala Glu Glu Arg Glu Ser Ala Arg Glu Glu Glu Glu Ala Glu
     355          360         365

Gln Glu Arg Arg Gly Gly Glu Glu Arg Val Gly Glu Glu Asp Glu Glu
     370          375         380

Ala Ala Glu Ala Glu Ala Glu Ala Glu Glu Ala Glu Arg Ala Arg Gln
385          390         395         400

Asn Ala Leu Leu Phe Ala Glu Glu Glu Asp Gly Glu Ala Gly Ala Glu
                405               410               415

Asp Lys Arg Ser Gln Glu Glu Thr Pro Gly His Arg Arg Lys Glu Ala
            420               425               430

Glu Gly Thr Glu Glu Gly Gly Glu Glu Glu Asp Asp Glu Glu Met Asp
            435               440               445

Pro Gln Thr Ile Asp Ser Leu Ile Glu Leu Ser Thr Lys Leu His Leu
        450               455               460

Pro Ala Asp Asp Val Val Ser Ile Ile Glu Glu Val Glu Glu Lys Arg
465               470               475               480

Lys Arg Lys Lys Asn Ala Pro Pro Glu Pro Val Pro Pro Pro Arg Ala
            485               490               495

Ala Pro Ala Pro Thr His Val Arg Ser Pro Gln Pro Pro Pro Pro Ala
            500               505               510

Pro Ala Pro Ala Arg Asp Glu Leu Pro Asp Trp Asn Glu Val Leu Pro
        515               520               525

Pro Trp Asp Arg Glu Glu Asp Glu Val Tyr Pro Pro Gly Pro Tyr His
        530               535               540

Pro Phe Pro Asn Tyr Ile Arg Pro Arg Thr Leu Gln Pro Pro Ser Ala
545 550 555 560

Leu Arg Arg Arg His Tyr His His Ala Leu Pro Pro Ser Arg His Tyr
565 570 575

Pro Gly Arg Glu Ala Gln Ala Arg Arg Ala Gln Glu Glu Ala Glu Ala
580 585 590

Glu Glu Arg Arg Leu Gln Glu Gln Glu Glu Leu Glu Asn Tyr Ile Glu
595 600 605

His Val Leu Leu Arg Arg Pro
610 615

<210> 10
<211> 189
<212> DNA
<213> Artificial

<220>
<223> DNA encoding amino acid sequence of SEQ ID NO: 1

<220>
<221> CDS
<222> (1)..(189)

<400> 10
aca ctg cag ccg ccc tcg gcc ttg cgc cgc cgc cac tac cac cac gcc        48
Thr Leu Gln Pro Pro Ser Ala Leu Arg Arg Arg His Tyr His His Ala
1               5                   10                  15

```
ttg ccg cct tcg cgc cac tat ccc ggc cgg gag gcc cag gcg cgg cgc      96
Leu Pro Pro Ser Arg His Tyr Pro Gly Arg Glu Ala Gln Ala Arg Arg
                20                  25                  30

gcg cag gag gag gcg gag gcg gag gag cgc cgg ctg cag gag cag gag     144
Ala Gln Glu Glu Ala Glu Ala Glu Glu Arg Arg Leu Gln Glu Gln Glu
            35                  40                  45

gag ctg gag aat tac atc gag cac gtg ctg ctc cgg cgc ccg tga        189
Glu Leu Glu Asn Tyr Ile Glu His Val Leu Leu Arg Arg Pro
        50                  55                  60
```

<210> 11
<211> 93
<212> DNA
<213> Artificial

<220>
<223> DNA encoding amino acid sequence of SEQ ID NO: 2

<220>
<221> CDS
<222> (1)..(93)

<400> 11
```
aca ctg cag ccg ccc tcg gcc ttg cgc cgc cgc cac tac cac cac gcc      48
Thr Leu Gln Pro Pro Ser Ala Leu Arg Arg Arg His Tyr His His Ala
1               5                   10                  15

ttg ccg cct tcg cgc cac tat ccc ggc cgg gag gcc cag gcg tga         93
Leu Pro Pro Ser Arg His Tyr Pro Gly Arg Glu Ala Gln Ala
                20                  25                  30
```

<210> 12
<211> 75
<212> DNA
<213> Artificial

<220>
<223> DNA encoding amino acid sequence of SEQ ID NO: 3

<220>
<221> CDS
<222> (1)..(72)

<400> 12
aca ctg cag ccg ccc tcg gcc ttg cgc cgc cgc cac tac cac cac gcc          48
Thr Leu Gln Pro Pro Ser Ala Leu Arg Arg Arg His Tyr His His Ala
1               5                   10                  15

ttg ccg cct tcg cgc cac tat ccc tga                                      75
Leu Pro Pro Ser Arg His Tyr Pro
            20

<210> 13
<211> 42
<212> DNA
<213> Artificial

<220>
<223> DNA encoding amino acid sequence of SEQ ID NO: 4

<220>
<221> CDS
<222> (1)..(42)

<400> 13
cac tac cac cac gcc ttg ccg cct tcg cgc cac tat ccc tga                   42

His Tyr His His Ala Leu Pro Pro Ser Arg His Tyr Pro
1                   5                   10

<210> 14
<211> 19
<212> PRT
<213> Rattus norvegicus

<400> 14

His Phe His His Ala Leu Pro Pro Ala Arg His His Pro Asp Leu Glu
1                   5                   10                  15

Ala Gln Ala

<210> 15
<211> 51
<212> PRT
<213> Rattus norvegicus

<400> 15

His Phe His His Ala Leu Pro Pro Ala Arg His His Pro Asp Leu Glu
1                   5                   10                  15

Ala Gln Ala Arg Arg Ala Gln Glu Glu Ala Asp Ala Glu Glu Arg Arg
                20                  25                  30

Leu Gln Glu Gln Glu Glu Leu Glu Asn Tyr Ile Glu His Val Leu Leu
                35                  40                  45

His Arg Pro
50

<210> 16
<211> 62
<212> PRT
<213> Rattus norvegicus

<400> 16

Thr Leu Gln Pro Pro Ala Ser Ser Arg Arg Arg His Phe His His Ala
1               5                   10                  15

Leu Pro Pro Ala Arg His His Pro Asp Leu Glu Ala Gln Ala Arg Arg
            20                  25                  30

Ala Gln Glu Glu Ala Asp Ala Glu Glu Arg Arg Leu Gln Glu Gln Glu
            35                  40                  45

Glu Leu Glu Asn Tyr Ile Glu His Val Leu Leu His Arg Pro
        50                  55                  60

<210> 17
<211> 13
<212> PRT
<213> Artificial

<220>
<223> consensus sequence of VGF derived peptide

```
<220>
<221>  MISC_FEATURE
<222>  (2)..(2)
<223>  Xaa is any amino acid


<220>
<221>  MISC_FEATURE
<222>  (9)..(9)
<223>  Xaa is any amino acid


<220>
<221>  MISC_FEATURE
<222>  (12)..(12)
<223>  Xaa is any amino acid


<400>  17


His Xaa His His Ala Leu Pro Pro Xaa Arg His Xaa Pro
1               5                   10




<210>  18
<211>  11
<212>  PRT
<213>  Artificial


<220>
<223>  consensus sequence of VGF derived peptide


<220>
<221>  MISC_FEATURE
<222>  (6)..(8)
<223>  Xaa is any amino acid


<400>  18
```

Thr Leu Gln Pro Pro Xaa Xaa Xaa Arg Arg Arg
1           5               10


<210> 19
<211> 6
<212> PRT
<213> Artificial


<220>
<223> consensus sequence of VGF derived peptide


<220>
<221> MISC_FEATURE
<222> (1)..(2)
<223> Xaa is any amino acid


<400> 19


Xaa Xaa Glu Ala Gln Ala
1           5



<210> 20
<211> 38
<212> PRT
<213> Artificial


<220>
<223> consensus sequence of VGF derived peptide


<220>
<221> MISC_FEATURE
<222> (1)..(2)
<223> Xaa is any amino acid

```
<220>
<221> MISC_FEATURE
<222> (14)..(14)
<223> Xaa is any amino acid


<220>
<221> MISC_FEATURE
<222> (36)..(36)
<223> Xaa is any amino acid


<400> 20


Xaa Xaa Glu Ala Gln Ala Arg Arg Ala Gln Glu Glu Ala Xaa Ala Glu
1               5                   10                  15


Glu Arg Arg Leu Gln Glu Gln Glu Glu Leu Glu Asn Tyr Ile Glu His
                20                  25                  30


Val Leu Leu Xaa Arg Pro
                35



<210> 21
<211> 24
<212> PRT
<213> Artificial


<220>
<223> peptide based on rat VGF sequence and corresponding to peptide 2


<220>
<221> MOD_RES
<222> (24)..(24)
<223> AMIDATION
```

<400> 21

Thr Leu Gln Pro Pro Ala Ser Ser Arg Arg Arg His Phe His His Ala
1                   5                   10                  15

Leu Pro Pro Ala Arg His His Pro
                    20


<210> 22
<211> 13
<212> PRT
<213> Artificial


<220>
<223> peptide based on rat VGF sequence and corresponding to peptide 1

<220>
<221> MOD_RES
<222> (13)..(13)
<223> AMIDATION


<400> 22

His Phe His His Ala Leu Pro Pro Ala Arg His His Pro
1                   5                   10


<210> 23
<211> 30
<212> PRT
<213> Artificial


<220>

<223> consensus amino acid sequence of VGF derived peptide

<220>
<221> MISC_FEATURE
<222> (6)..(6)
<223> Xaa is any amino acid

<220>
<221> MISC_FEATURE
<222> (28)..(28)
<223> Xaa is any amino acid

<400> 23

Ala Gln Glu Glu Ala Xaa Ala Glu Glu Arg Arg Leu Gln Glu Gln Glu
1               5                   10                  15

Glu Leu Glu Asn Tyr Ile Glu His Val Leu Leu Xaa Arg Pro
            20                  25                  30

<210> 24
<211> 32
<212> PRT
<213> Artificial

<220>
<223> consensus sequence of VGF derived peptide

<220>
<221> MISC_FEATURE
<222> (6)..(8)
<223> Xaa is any amino acid

<220>

```
<221>  MISC_FEATURE
<222>  (13)..(13)
<223>  Xaa is any amino acid


<220>
<221>  MISC_FEATURE
<222>  (20)..(20)
<223>  Xaa is any amino acid


<220>
<221>  MISC_FEATURE
<222>  (23)..(23)
<223>  Xaa is any amino acid


<220>
<221>  MISC_FEATURE
<222>  (25)..(26)
<223>  Xaa is any amino acid


<400>  24
```

Thr Leu Gln Pro Pro Xaa Xaa Xaa Arg Arg Arg His Xaa His His Ala
1               5                   10                  15

Leu Pro Pro Xaa Arg His Xaa Pro Xaa Xaa Glu Ala Gln Ala Arg Arg
                20                  25                  30

```
<210>  25
<211>  30
<212>  PRT
<213>  Rattus norvegicus


<400>  25
```

Ala Gln Glu Glu Ala Asp Ala Glu Glu Arg Arg Leu Gln Glu Gln Glu
1                5                        10                    15

Glu Leu Glu Asn Tyr Ile Glu His Val Leu Leu His Arg Pro
            20                    25                    30

<210> 26
<211> 24
<212> PRT
<213> Homo sapiens

<220>
<221> MOD_RES
<222> (24)..(24)
<223> AMIDATION

<400> 26

Thr Leu Gln Pro Pro Ser Ala Leu Arg Arg Arg His Tyr His His Ala
1                5                        10                    15

Leu Pro Pro Ser Arg His Tyr Pro
            20

<210> 27
<211> 13
<212> PRT
<213> Homo sapiens

<220>
<221> MOD_RES
<222> (13)..(13)

<223> AMIDATION

<400> 27

His Tyr His His Ala Leu Pro Pro Ser Arg His Tyr Pro
1               5                   10

<210> 28
<211> 19
<212> PRT
<213> Homo sapiens

<400> 28

Asn Ala Pro Pro Glu Pro Val Pro Pro Pro Arg Ala Ala Pro Ala Pro
1               5                   10                  15

Thr His Val

<210> 29
<211> 20
<212> PRT
<213> Homo sapiens

<400> 29

Glu Glu Asp Glu Val Tyr Pro Pro Gly Pro Tyr His Pro Phe Pro Asn
1               5                   10                  15

Tyr Ile Arg Pro
                20

<210> 30
<211> 37
<212> PRT
<213> Homo sapiens

<400> 30

Asn Ala Pro Pro Glu Pro Val Pro Pro Pro Arg Ala Ala Pro Ala Pro
1               5                   10                  15

Thr His Val Arg Ser Pro Gln Pro Pro Pro Ala Pro Ala Pro Ala
            20                  25                  30

Arg Asp Glu Leu Pro
            35

<210> 31
<211> 38
<212> PRT
<213> Homo sapiens

<400> 31

Asn Ala Pro Pro Glu Pro Val Pro Pro Pro Arg Ala Ala Pro Ala Pro
1               5                   10                  15

Thr His Val Arg Ser Pro Gln Pro Pro Pro Ala Pro Ala Pro Ala
            20                  25                  30

Arg Asp Glu Leu Pro Asp
            35


<210>  32
<211>  75
<212>  PRT
<213>  Rattus norvegicus


<400>  32


Asn Ala Pro Pro Glu Pro Val Pro Pro Pro Arg Ala Ala Pro Ala Pro
1               5                   10                  15


Thr His Val Arg Ser Pro Gln Pro Pro Pro Ala Pro Ala Arg Asp
            20                  25                  30


Glu Leu Pro Asp Trp Asn Glu Val Leu Pro Pro Trp Asp Arg Glu Glu
            35                  40                  45


Asp Glu Val Phe Pro Pro Gly Pro Tyr His Pro Phe Pro Asn Tyr Ile
        50                  55                  60


Arg Pro Arg Thr Leu Gln Pro Pro Ala Ser Ser
65                  70                  75


<210>  33
<211>  129
<212>  PRT
<213>  Rattus norvegicus

<400> 33

```
Asn Ala Pro Pro Glu Pro Val Pro Pro Pro Arg Ala Ala Pro Ala Pro
1               5                   10                  15

Thr His Val Arg Ser Pro Gln Pro Pro Pro Ala Pro Ala Arg Asp
            20                  25                  30

Glu Leu Pro Asp Trp Asn Glu Val Leu Pro Pro Trp Asp Arg Glu Glu
            35                  40                  45

Asp Glu Val Phe Pro Pro Gly Pro Tyr His Pro Phe Pro Asn Tyr Ile
            50                  55                  60

Arg Pro Arg Thr Leu Gln Pro Pro Ala Ser Ser Arg Arg Arg His Phe
65                  70                  75                      80

His His Ala Leu Pro Pro Ala Arg His His Pro Asp Leu Glu Ala Gln
                85                  90                  95

Ala Arg Arg Ala Gln Glu Glu Ala Asp Ala Glu Glu Arg Arg Leu Gln
                100                 105                 110

Glu Gln Glu Glu Leu Glu Asn Tyr Ile Glu His Val Leu Leu His Arg
            115                 120                 125

Pro
```

<210> 34
<211> 60
<212> DNA
<213> Artificial

<220>
<223> DNA encoding amino acid sequence of SEQ ID NO: 28

<220>
<221> CDS
<222> (1)..(60)

<400> 34

```
aac gcc cct ccc gag ccc gtg ccg ccc ccc cgt gcc gcc ccc gcc ccc        48
Asn Ala Pro Pro Glu Pro Val Pro Pro Pro Arg Ala Ala Pro Ala Pro
1               5                   10                  15

acc cac gtc tga
Thr His Val
```

<210> 35
<211> 63
<212> DNA
<213> Artificial

<220>
<223> DNA encoding amino acid sequence of SEQ ID NO: 29

<220>
<221> CDS
<222> (1)..(63)

<400> 35

```
gag gag gac gag gtg tac ccg cca ggg ccg tac cac cct ttc ccc aac        48
Glu Glu Asp Glu Val Tyr Pro Pro Gly Pro Tyr His Pro Phe Pro Asn
1               5                  10                 15


tac atc cgg ccg tga
Tyr Ile Arg Pro
                20
```